# EUROPEAN PATENT SPECIFICATION

(11) **EP 0 594 763 B1**
(45) Date of publication and mention of the grant of the patent: **23.09.1998**
(21) Application number: 92916006.7
(22) Date of filing: 10.07.1992
(51) Int. Cl.: G01N 33/536, G01N 33/546, G01N 33/543, G01N 33/569, G01N 33/68, G01N 33/576

(54) **METHODS AND COMPOSITIONS FOR SIMULTANEOUS ANALYSIS OF MULTIPLE ANALYTES**
VERFAHREN UND ZUSAMMENSETZUNGEN FÜR DIE GLEICHZEITIGE ANALYSE EINER VIELZAHL VON ANALYTEN
PROCEDES ET COMPOSITIONS D'ANALYSE SIMULTANEE D'ANALYTES MULTIPLES

(30) Priority: 16.07.1991 US 731039
(43) Date of publication of application: 04.05.1994
(73) Proprietor: TRANSMED BIOTECH INCORPORATED, South San Francisco, CA 94080 (US)
(72) Inventor: LEHNEN, Brian, C., San Carlos, CA 94070 (US); CROTHERS, Stephan, D., Palo Alto, CA 94303 (US)
(74) Representative: Goldin, Douglas Michael
(86) International application number: PCT/US92/05799
(87) International publication number: WO 93/02360

(56) References cited:
- GB-A- 1 561 042
- US-A- 4 499 052
- US-A- 4 584 277
- US-A- 4 786 165
- US-A- 4 869 903
- US-A- 4 918 004
- US-A- 4 960 713
- US-A- 5 073 497
- CYTOMETRY, vol. 0, no. supplement 2, 4 September 1988, NEW YORK NY USA, page 19 XP000567971 M.J. FULWYLER ET AL.: "Immunoreactive bead (IRB) assay for the quantitative and simoultaneous flow cytometric detection of multiple soluble analytes."

## Description

### INTRODUCTION

### Technical Field

The present invention relates to a method and apparatus for use therein for the contiguous detection of multiple analytes in a biological sample using multiple complementary binding moieties. The invention is exemplified by the contiguous detection of human IgG, human chorionic gonadotropin and antibodies to recombinant HIV gp41, recombinant HIV p24, Hepatitis B core protein, and recombinant HTLV re-5.

### Background

The in vitro diagnostics industry has been seeking technologies which afford the contiguous discrete detection of multiple analytes. As used in this application, the term "contiguous" refers to assays carried out both in the same space and at the same time, in contrast to the term "simultaneous", which refers to assays carried out at the same time, but not necessarily in the same space. As an example, chlamydia and gonorrhea infections often are coincident in women. Collecting a specimen can be problematic so that a single assay which can detect both disease agents contiguously is desirable. Another example is the current interest in the simultaneous detection of HIV-1 and HIV-2 antibodies in blood. Using a contiguous assay, a positive finding indicates the presence of antibodies to either one or to both of the viruses but does not allow a determination of which antibody, specifically, is present. Such an assay technology has somewhat less diagnostic utility than a simultaneous discrete assay affords but offers a measure of convenience and cost effectiveness.

Another reason for the interest in the contiguous, discrete analysis of multiple analytes lies in the use of such technology in so called panel testing and screening assays. In panel testing, for a given specimen under diagnostic interrogation a number of different assays are ordered together for the purpose of diagnostic interpretation. In screening assays, the laboratory performs the same set of simultaneous assays on every specimen in an effort to determine if the specimen is positive for one or more of the analytes in the screen.

An example of the latter application is screening of blood in the blood bank where every unit of donated blood is screened for HIV, HBV, HTLV, and HCV. In either of these situations a reagent technology which affords the contiguous discrete analysis of multiple analytes in a single reagent would have obvious cost and convenience benefits. Presently this need for efficient screens or panels is accommodated using robotics where simultaneous multiple but separate assays are performed by a preprogrammed reagent and sample handling system. Such systems are expensive and can be unreliable. Examples of potentially useful panels which may be accommodated by the contiguous assay of the presently claimed invention include blood bank screen, hepatitis panel, AIDS screen, fertility panel, thyroid panel, tumor markers, infectious disease, allergy panel, anemia panel, auto antibody panel, microbiological antigens or antibodies, pesticide analytes, drugs of abuse or toxic chemical analyses amenable to immunoassay.

It is therefore of interest to develop the capability to allow the medical diagnostician to perform contiguous assays of many analytes. It is also of interest to develop a low cost apparatus for carrying out multiple contiguous analyses.

### Relevant Literature

Recent review articles trace the history of the technological evolution of concepts for multiple simultaneous immunoassays (Fulwyler and McHugh, Methods in Cell Biology 33:613-619,1990 and McHugh, Immunochemica 5: No. 1, 1991). These concepts trace back to UK patent #1561042 assigned to Coulter Inc by Fulwyler in 1976. In addition, Shapiro, Practical Flow Cytometry, 3d Ed., (1988) Ch. 7: 115-198 discusses various parameters which can be measured in a fluorescence flow cytometer. Gosling, "A Decade of Development in Immunoassay Methodology," Clin. Chem. 36:1408-1427 (1990), discusses immunoassays in general. Some of these methods involve the use of separately identifiable subpopulations of immunoreactive microspheres which can be detected in a flow cytometer. Related US patents include USPN 4,499,052, USPN 4,526,276, USPN 4,717,655. Detection of cells using a flow cytometer has also been disclosed in the following US patents, USPN 4,988,619, USPN 4,987,086, USPN 4,859,584, USPN 4,783,401 and USPN 4,762,701.

Simultaneous determination of multiple immunoreactive analytes employs the use of fluorescent immunoassays (FIAs) in which the capture matrix, or solid phase, generally is a microsphere of known diameter. For example a 10 micron diameter microsphere can be coated with an antigen A. When this coated microsphere is exposed to a fluid which contains antibodies to antigen A, these antibodies will specifically bind to the coated microsphere. Next a reagent which contains a fluorochrome labelled antibody which will specifically bind to antibody A is added. Thus an antibody "sandwich" arises when antibody A is present in the sample such that the fluorochrome is now bound to the microsphere via the specific antibody antigen pairing. This is an example of an "antibody capture" FIA. Alternatively the microsphere can be coated with certain antibodies and thus selectively capture antigens which might be present in the sample in an "antigen capture" immunoassay. In this manner the effective fluorescence of the 10 micron diameter microsphere is determined by how many specific antigens or antibodies are captured, i.e., are present in the sample.

Since the flow cytometer can co-determine particle size and fluorescence intensity, several different immunoassays can be contiguously performed on the same sample by coating differing sizes of microspheres with different analytes, i.e., one immunoassay per microsphere size (see Fulwyler *supra).* McHugh *supra,* discusses the use of multiple microsphere sizes using "gating," and demonstrates a 4-analyte identification by this method. Furthermore, McHugh postulates that up to 9 gates may be used depending upon the variation in microspheres in each size category commercially available. Fulwyler *supra,* postulates that the use of microspheres containing a non-interfering fluorescent dye can be used to double the number of analytes identifiable. All applications of this technology to the contiguous discrete detection of multiple analytes rely upon the use of flow cytometry to discriminate one analyte from another based upon the differing sizes of the microspheres employed. Thus the number of different analytes which can be so differentiated is determined by the particle size resolution capabilities of FACS technology and upon the number of different sizes of microspheres which can be reliably manufactured to a precise size, as well as the number of non-interfering fluorescent dyes which can be distinguished.

Fulwyler *et al,* Cytometry, Vol. O, Supp. 2, p.19, 1988, refers to an assay for the quantitative and simultaneous flow cytometric detection of multiple soluble analytes.

### SUMMARY OF THE INVENTION

The subject invention provides for the performance of contiguous assays to detect multiple analytes of interest in a sample using a reagent made up of multiple discrete ligant complexes. Each ligand complex comprises a ligand attached to a plurality of solid supports of a shape which can be detected in a flow cytometer. The solid support to which each discrete ligand is attached may be of the same size for each ligand, or of different sizes. Each analyte and its ligand, or complementary binding moiety, comprise first and second members of a specific binding pair (msbp), respectively. The method includes the steps of combining to form a reagent, known proportions of each ligand complex, contacting the reagent with a sample for a time and under conditions whereby specific binding pairs are formed on the solid supports, detecting a preselected number of supports and relating the presence of particular analytes of interest to the formation of specific binding pairs associated with each of the discrete ligand complexes. The specific binding pairs are detected by means of a detectable label. The methods of the subject invention greatly extend the number of contiguous analyses tha t may be performed on a single sample and find particular application in screens, panels and combination tests of all types. Generally, at least five analytes, more usually three to five discrete analytes, can be identified in a single assay without the use of discriminators, such as changes in bead size or the use of multiple fluorescers and detectors.

The method of analysis of the data obtained includes the steps of collecting data by way of the detectable label for a predetermined number of solid supports as a function of the intensity of the label for each of the preselected proportions of complementary binding moieties. The relative intensity of the label can be used to indicate the presence or absence of an analyte of interest in the sample.

The apparatus of the subject invention is for detecting fluorescence from a sample containing solid supports flowing in a liquid path. The apparatus includes means for detecting at least one fluorochrome capable of excitation and means for accumulating the total number of particles and/or total fluorescence associated with each of the several subpopulations of solid supports. Means of recording the data collected is provided so that the relative intensity of fluorescence collected for a predetermined number of solid supports may be associated with each of the preselected ratios of binding moieties. The apparatus provides an alternative to traditional flow cytometers for particle analysis, and finds particular application with the method of the subject application.

### BRIEF DESCRIPTION OF THE DRAWINGS

Figure 1 is a graphic representation of a two parameter contour plot or gate showing the union of side scatter and forward scatter of three different size microsphere populations.

Figure 2 is a graphic representation of a single parameter forward light scatter histogram of the three microsphere populations represented in Figure 1.

Figure 3 is a graphic representation of a fluorescence histogram of microspheres stained with fluorescein-labeled goat f(ab') anti-human IgG (Fc specific) (hereinafter "FITC-aIgG").

Figure 4 is a schematic representation of a cytometric apparatus for detecting all of side scatter, forward scatter, and fluorescence or two or more of these characteristics in combination of individual reacted particles moving in a flow path from the reaction vessel.

Figure 5 is a schematic representation of a flow apparatus for detecting and analyzing fluorescence of individual reacted particles moving in a flow path from the reaction vessel.

Figure 6 is a flow chart showing the steps of the algorithm for histogram analysis.

Figure 7 shows the analysis of a specific histogram.

### BRIEF DESCRIPTION OF THE SPECIFIC EMBODIMENTS

In accordance with the subject invention compositions, methods and an apparatus are provided for the performance of contiguous discrete assays of multiple analytes of interest in a sample, particularly a biological sample. The analytes may be polypeptides such as antigens, antibodies, nucleic acids, haptens, carbohydrates or combinations thereof. The method relies upon the use of a mixture of known proportions of complementary binding moieties to each analyte of interest; each analyte and its complementary binding moiety comprise first and second members of a specific binding pair (msbp), respectively. Each complementary binding moiety is attached to a solid support, generally a microsphere, that may be detected by flow cytometry techniques to produce a reagent. The identification of the analyte does not depend upon the size or other physical characteristic of the solid support to which the complementary binding moiety is bound. Therefore, all complementary binding moieties may be bound to solid supports of the same average size, although more than one size may be used if desired or if the number of analytes to be detected is particularly large.

The subject invention allows the performance of contiguous discrete analysis of multiple analytes without reliance upon microsphere size discrimination. Further, it allows the contiguous performance of a larger number of assays than can be accomplished with the conventional use of FACS and immunoreactive microspheres as described heretofore. At least four, generally five or six analytes, and possibly up to 15 analytes may be assayed contiguously using a single microsphere size and/or a single fluorochrome; i.e., without the use of discriminaters. Adding multiple microsphere sizes or other physical discriminators or the use of additional fluorochromes may be used to increase the number of analytes assayed contiguously. Thus, the methods of the subject invention greatly extend the number of contiguous analyses that may be performed on a single sample and find particular application in screens, panels and combination tests of all types. Coupled with techniques known to those skilled in the art, the method claimed has the power to analyze up to 15 analytes times 8 size discriminators times 3 fluorescent discriminators, or 360 contiguous assays. A more practical estimate of power is 8 analytes times 5 size discriminators times 2 fluorescent discriminators, or 80 analytes assayed contiguously. The maximum number of analytes may limited by the data handling and storage by the computer software associated with the apparatus; these limits can be overcome by changing parameters within the software, and employing a computer with greater data handling and storage capabilities than that described. The maximum number of analytes also may be limited by the amount of cross-reactivity, non-specific binding and interferences which can be overcome by optimizing the analyte reagents.

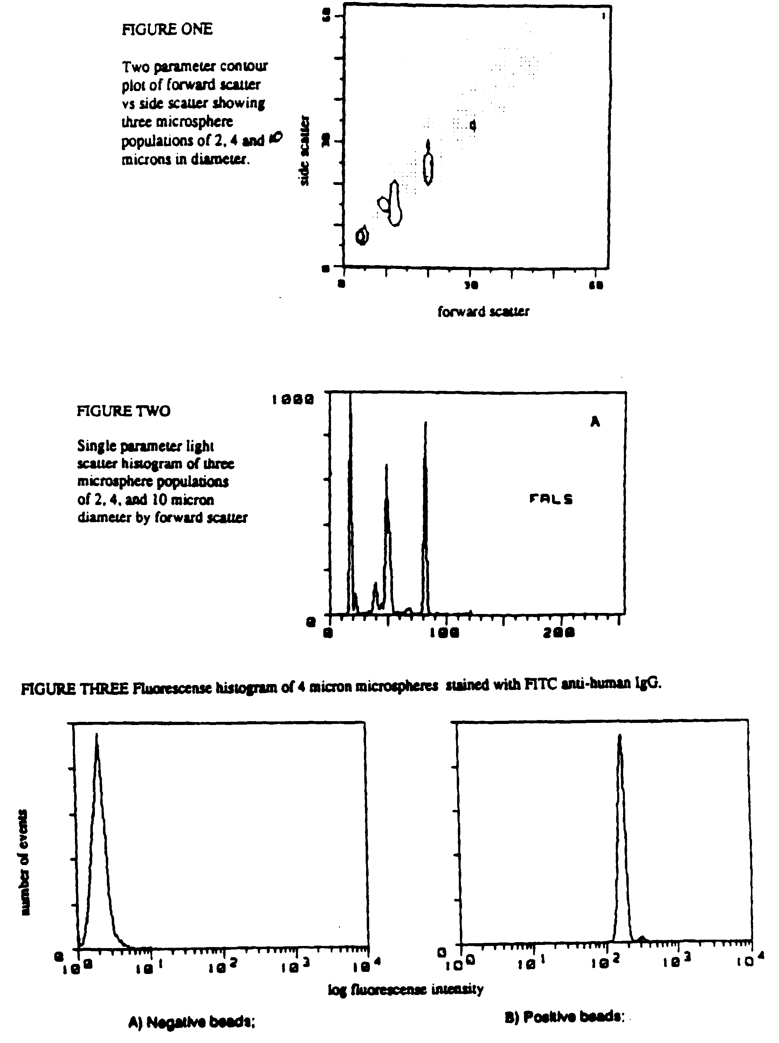

In a conventional assay, the results of the performance of immunoassays on microspheres in FACS or "flow" instrumentation are interpreted in the following way: When right angle light scattering of incident light is plotted against forward angle light scattering of incident light, the resultant is a series of derivative functions of microsphere size as shown below in Figure 1. Each of the profiles in this two parameter light scatter profile defines a microsphere population of a particular size. Alternatively, in a single parameter profile in which only forward angle light scattering (FALS) is plotted, the number of events, i.e., the number of microspheres of that size which passed through the light path, can be plotted as shown in Figure 2. Computer aided reduction of these data is used to "gate" the data such that only the data from microspheres of a particular size range is viewed, i.e., only the number of events at certain FALS values are shown. By "gate" is intended that only a population(s) of microspheres of a size within a specific size range are considered in analysis of the data. McHugh *supra,* has demonstrated that adequate separation of microsphere populations can be achieved using FALS only.

When analyzing the data from assays done using microspheres of varying sizes, the gating technique above is used to focus upon one microsphere size population at a time. Within this "gate" the fluorescence intensity of the microspheres of that size range is determined by the degree of binding of the analyte in the immunosandwich. These data are output in the form of a histogram as shown in Figure 3. Here the number of events, i.e., the number of microspheres within that size range which passed through the light path, is plotted against the fluorescence intensity of those microspheres on a log scale.

If the sample were a serum sample, for example, a positive serum would reveal a "peak" relatively further towards the right on the X axis than would a negative serum. However even a negative serum will have a representative peak because of nonspecific binding of the fluorochrome labelled second antibody to the microsphere. The position of the peak on the log scale X axis (fluorescence intensity) is reported as the "mean channel fluorescence(mcf)". Thus the mcf value of a histogram peak is a measure of the degree of reactivity, ie presence of the analyte, wherein the analyte is specifically identified by the "gate" in which the histogram lies. Of course this gate is determined by the microsphere size as shown in Figures 1 and 2. The area under the histogram peak has not previously been used to convey analytical information.

Using this methodology, wherein each analyte is detected via an immunoassay performed on a pre-selected and unique size microsphere and wherein each microsphere size determines the electronic gate within which fluorescence intensity can be determined, contiguous discrete immunoassays for multiple analytes have been performed since 1974. This methodology is limited by the number of gates which can be resolved and the numbers and kinds of microspheres which are available. Scillian et al (BLOOD 73: 2041 - 2048, 1989) reports four simultaneous antibody capture assays.

Two issues are noteworthy. One is the standard methods by which investigators in this field configured these multiple microsphere reagents and the other is the way in which the gated histogram data is reported. For the gated histogram data (see Figure 3), the Y-axis is often labelled "RELATIVE number of events". The actual enumeration of the Y-axis values could be expressed as ABSOLUTE numbers of events. Further, it is standard practice to mix the multiplex reagents such that the number of microspheres of each size is the same as for all other sizes in the common reagent, i.e. they are mixed in approximately equal proportions or even if they are mixed in different proportions, significance is not realized. This practice also leads to an internal consistency in the size and shape of the peaks in the histogram which can mask certain properties of this technology.

In the subject method, it is possible to screen contiguously for multiple analytes, without relying upon the physical characteristics of the microspheres or on using multiple fluorochromes. Identification of the presence or absence of an analyte in the sample instead may be made by preparing a mixture containing a known proportion of each subpopulation of ligand complex. A ligand complex is defined as a plurality of solid supports of a shape capable of being detected by flow cytometry techniques to which is bound a complementary binding moiety capable of binding specifically to a single analyte of interest in a biological sample. The composition thus will comprise a mixture of multiple discrete reagents capable of detection by flow cytometry techniques. The proportion that each sub-population constitutes of the total composition must be known because it later is used in identifying an analyte of interest.

Once the total composition has been prepared, it is then combined with a sample using appropriate buffers, temperatures, and time periods so that specific binding pairs may form. The specific binding pairs are contacted with a labeling agent, and a predetermined number of solid supports are then detected using flow cytometry techniques. The relative intensity of label associated with the solid supports is collected and the data plotted as a histogram of number of events versus relative label intensity. The data are then analyzed to identify analytes present in the sample based upon the proportion of each sub-population of reagents used in the assay.

In some instances, it may be advantageous to use properties so that any combination of two or more subpopulations of binding moieties is also unique. For example, if there are three analytes of interest, three unique nonadditive proportions of complementary binding moieties could be 1:2:7. If a specified number of solid supports is then counted, for example 5000, then the number of solid supports associated with each subpopulation, based upon the proportion present in the original reagent, would be 500, 1000, and 3500. If all three analytes were absent from the sample, then a population of 5000 solid supports would be identified having background fluorescence. If two of the analytes were present in the sample and were bound by the binding moieties which were present at 10% and 20% of the original reagent composition, then two populations could be identified, one representing the 70% subpopulation for which no analyte is present and together one representing the remaining two subpopulations which represent 30% of the mixture. Where the two analytes are present in differing amounts, then three separately identifiable subpopulations would be observed based upon the relative intensity of label detected. This is illustrated further below. The assay method of the invention can be used to detect any analyte that is capable of binding to a complementary binding moiety.

Generally, the analyte will be a polypeptide, protein, carbohydrate, polysaccharide, nucleic acid, drug of abuse or a metabolite of such a drug or combinations thereof. Techniques are known to those skilled in the art for the methodology for assaying for any of the recited types of analytes. See, for example, Fulwyler & McHugh, *supra;* Shapiro, Practical Flow Cytometry, Ch. 7: 115-198 (1988); Sigma 1992 Immunochemicals Catalog, p. 285 (insoluble proteins A & G); Cappel/Orgnanon/Technica 1991 Catalog (immunochemical reagent); Rylatt *et al.,* "A rapid whole blood immunoassay system, The Medical Journal of Australia, 152:75-77 (1990) (cross-linked fibrin degradation products, Hepatitis surface antigen, HIV antibody, theophylline, and digoxin); Brinchman *et al.,* "Direct immunomagnetic quantification of lymphocyte subsets in blood," Clin. exp. Immunol., 71:182-186 (1980) (CD2, CD4, CD8, CD19 analytes on whole cells); Gosling, *supra;* Saunders *et al.,* "Flow-cytometric competitive binding assay for determination of Actinomycin-D concentrations, "Cytometry, 11:311-313 (1990) (drug, DNA); Bangs, "Latex Agglutination Tests, " American Clinical Laboratory News Ed., (1988) (lists of analytes); Cuatrecasas, PNAS, 61:633 (1963) (coupling-affinity resins, agarose).

For the most part, the analytes detected or quantified in accordance with the present invention will preferably have a molecular weight of at least about 5,000, more usually at least about 10,000. Polypeptides of interest will generally be from about 5,000 to about 5,000,000 molecular weight, more usually from about 10,000 to 1,000,000 molecular weight. Where the analyte is a nucleic acid molecule, the molecule will generally range from about 12 nucleotides to about 2x10⁶ nucleotides. The nucleic acid sample can involve DNA, which can be chromosomal or extrachromosomal, e.g., plasmids, viruses, synthetic constructs, or the like, or RNA, such as messenger RNA, transfer RNA, ribosomal RNA, viruses, or the like. The nucleic acid sequences can involve structural genes on translated regions, regulatory regions, introns, exons, and the like.

Analytes for detection or quantitative with the present invention can also have molecular weight of less than about 5,000. Examples of suitable analytes with a molecular weight of less than 5,000 include small polynucleotides, small polypeptide hormones, steroid hormones, cholesterol, drugs, drugs of abuse and toxins. The complementary binding moieties are components capable of specific binding to the particular analyte of interest. The binding between the binding moieties and the analyte of interest is preferably non-covalent. Suitable binding moieties preferably have higher affinity and specificity for the analyte than for the other components in a sample for analysis. Suitable binding moieties can be of a variety of molecular categories including antibodies, in particular, and preferably, monoclonal antibodies specific for a portion of the analyte; binding proteins that naturally bind to the analyte, e.g. lectins for analytes comprising a carbohydrate portion; and ligand receptors when the analyte comprises a complementary ligand. Some examples of binding moieties include: anti-HBsAg antibody either animal derived polyclonals or mammalian monoclonals, for example, mouse, rat or human; HIV gp 41 purified from the virus or derived by recombinant DNA technology; concanavalin A; jaclin; and CD4 from human T lymphocytes. Techniques for preparing solid supports which comprise the binding moiety are well known to those skilled in the art. See Fulwyler, *supra;* Shapiro, *supra;* Sigma, *supra,* Cappel, *supra;* Rylatt, *et al., supra;* Brinchman, *et al., supra;* Gosling, *supra;* Saunders, *et al., supra;* Bangs, *supra;* Cuatracasas, *supra.* See also Cantarero, *et al.,* "The adsorptive characteristics of proteins for polystyrene and their significance in solid-phase immunoassays," Analytical Biochem., 105:375-382 (1980); Holodniy, "Detection and Quantification of human immunodeficiency virus RNA in patient serum by use of the polymerase chain reaction, "J Infectious Dis., 163:862-866(1991); Bangs Laboratories Technical Reference No. 21, "Particles used in DNA probes" Methods for the production of antibodies or monoclonal antibodies to be used as binding moieties in the subject invention are known in the literature. See, e.g., U.S. Patent No. 4,574,116 and the references cited therein. Alternatively, monoclonal antibodies and/or binding fragments can be purchased commercially.

When the analyte is a nucleic acid, the binding moiety can be ssDNA, RNA, or any other natural or synthesized single stranded nucleic acid. Alternatively the binding moiety could be a non-nucleic acid molecule which recognizes a specific nucleotide sequence, such as an antibody or specific DNA binding protein on the analyte.

When the binding moiety is a nucleic acid molecule, the moiety will usually comprise at least 8 nucleotides, more usually 10 nucleotides, and preferably at least about 12 nucleotides. The size of the binding moiety will vary with the nature of the analyte, the amount of analyte in the sample, and the conditions employed in the detection process. The nucleic acid sequences for use in a binding moiety can be provided by isolation from a natural source, synthesis, or other means known in the art, as described in Holodniy, *supra,* and Bangs Laboratories Technical Reference No. 21, *supra.*

The sample can be subjected to prior preparation or can be used without prior treatment. See Holodniy, *supra.* Sample preparation is a property of the sample, not of the immunoassay. The protocol for the assay can accommodate the contiguous detection of both antibodies and antigens. For example, one subpopulation of microspheres is coated with antigen and another population is coated with antibodies derived from an animal species such as goat, mouse, rat, rabbit, etc. The second reagent contains fluorescent labelled antibodies of at least two types. In the case where the sample is of human origin the second reagent includes antihuman antibodies which selectively bind with any analyte antibody which has bound to the antigen coated microsphere above and, for example, goat antibody which selectively binds the analyte in the sample which has bound to the antibody coated microsphere. The two antibodies in the second reagent will not cross react because they are idiotypically distinct. The labelled antibodies do not need to be in any particular proportion in the reagent so long as both are present in excess.

In the situation in which the analyte in the sample is capable of binding with the binding moieties, no prior sample preparation is generally necessary. In the situation in which the analyte in the sample is not immediately capable of binding with the binding moieties, prior sample preparation will be necessary. For example, where the analyte is double stranded nucleic acid and the binding moieties are complementary nucleic acid strands, it will be necessary to treat the sample to denature the double-stranded molecules before mixing with the binding moiety. Denaturation can be achieved most readily by subjecting the sample to high temperature, generally from about 90°C to about 100°C for about 3 to about 15 minutes. Other means for denaturation can be used such as treating the sample with alkaline solutions or concentrated solutions of formamide or through use of other procedures known in the art.

The solid supports preferably will comprise particles of microspheres having an average diameter of about 0.25 micron to about 100 micron, most preferably about 2 micron to about 15 micron where cost may be a factor (small microspheres are less expensive than large microspheres). The size of the microspheres is not critical to the practice of the subject invention. Suitable materials for the solid supports include organic polymers, both naturally occurring and synthetic, such as polysaccharides, styrene polymers, polyacrylates, e.g., polyacrylamide, hydroxyethyl polymethacrylates, glass, ceramic, carbon, polyvinyl chloride, protein, and the like. Styrene polymers include polystyrene, polymers containing aromatic moieties, and higher aromatic compounds such as naphthalene, anthracene, etc. The solid supports preferably consist of a latex compound. For examples of the use of solid supports known to those skilled in the art, see, for example, Montagne *et al.,* "Polyacrylic microspheres as a solid phase for microparticle enhanced nephelometric immunoassay, "J Immunoassay, 12:165-183 (1991); Lisi *et al.,* "A fluorescence immunoassay for soluble antigens employing flow cytometric detection," Clinica Acta, 120:171-179 (1982); Saunders *et al.,* 'Amplified Flow Cytometric Separation-Free Fluorescence Immunoassays," Clin. Chem., 31:2020-2023 (1985); Wilson *et al.,* "A new microsphere-based immunofluorescence assay using flow cytometry, "J Immuno. Meth., 107:225-230 (1988).

Following binding of the complementary binding moiety to the solid supports, the compositions for use in the assay protocol are prepared. The compositions include multiple subpopulations of solid supports, typically microspheres. To prepare the compositions, the microspheres must be counted by one of several methods which include: manual counting using a microscope and a hemocytometer; using a commercial particle counter, which generally measures the particle number using electrical impedance changes; using a rate or volume-calibrated flow cytometer; or quantitating light scatter generated by particles in a spectrophotometer.

The various solid supports can be functionalized or non-functionalized, preferably functionalized for covalent bonding to the binding moiety. When the linking element is a polymeric material, various procedures are known in the art for the activation of polymer surfaces and the attachment of immunoglobulins, glycoproteins, saccharide-containing organic molecules, and polynucleotides. See U.S. Patent Nos. 4,419,444; 4,775,619; 3,956,219; and 3,860,486 as well as European Patent Application No. 84308143.1 and Scouten, W.H. (ed.) Solid Phase Biochemistry, Analytical and Synthetic Aspects (1983), Wiley & Sons, New York. Bifunctional organic linking groups can be used to attach the complementary binding moiety to the solid support. Examples of such groups, which are well known in protein chemistry, include dialdehydes, such as glutaraldehyde, and diamines, such as 1,6-diaminohexane. Such bifunctional organic linking groups are preferably used when the binding moiety is sufficiently large to prevent complementation.

The protocol for the assay can be varied widely, depending upon the system being employed, the sensitivity of the assay, the speed with which the assay is to be carried out, the nature of the analyte, and the like. The binding moiety and sample are combined together under appropriate conditions to allow for binding. The reagents can be combined concomitantly or added sequentially. Where the order is sequential, the reaction mixture of the sample, the binding moiety and buffer is preferably incubated for about 5 to 90 minutes, usually about 30 minutes, before washing the mixture and the addition of labelled reagent. A fluorochrome label is generally and preferably used for such a purpose by providing a detectable label upon excitation of the fluorochrome with energy of the appropriate wavelength. The amount of analyte in the sample can then be determined by comparing the amount of detectable label to that formed in the presence of a known amount of analyte.

The fluorochrome typically and preferably employed results in a change in the amount of light absorbance (optical density) or emission of the assay medium when excited by energy of the appropriate wavelength. Preferred fluorochromes include but are not limited to fluorescein isocyanate, phycoerythrin, Duochrome, allophycocyanin, PE Tandem, ultralite 700 and Texas red and are commercially available. The fluorochromes are generally used at a fluorescent molecule to protein (F:) molar ratio of about 1 to about 8. Other fluorochromes can be used to increase the sensitivity of an assay by using a fluorochrome with a higher signal to noise. See Shapiro, *supra;* Kronick *et al.,* "Immunoassay Techniques with Fluorescent Phycobifiprotein conjugates," Clin.Chem., 29/9:1582-1586 (1983); Canterero *et al., supra;* Gosling, *supra;* Allain *et al.,* "Automated Fluoroimmunoassay of Theophylline and Valproic Acid by Flow-Injection Analysis with Use of HPLC Instruments," Clin.Chem., 35:469 (1989); Goding, "Conjugation of antibodies with fluorochromes: modifications to the standard methods, "J Immun. Meth., 13:215 (1976).

The present invention contemplates the use of other light emitting moieties rather than fluorochromes such as phosphorescent, chemiluminescent or bioluminescent moieties. When these light emitting labels are used as dyes, a laser is not required so that the derivative apparatus employs a fluidics device and a light detector with the appropriate electronics.

The sample is combined with the mixture containing the known proportions of multiple subpopulations of immobilized complementary binding moieties and incubated for a time sufficient for specific binding pairs to form. The amount of sample that can be used in conjunction with the present invention depends, among other things, upon the concentration of the analyte, the nature of the sample, and the sensitivity of the assay. The time required for the completion of the desired reactions may vary depending on the particulars of the assays. The time required for hybridization or binding depends on the concentration and sequence complexity of the reagents and the analyte, as well as on the assay temperature, solvent, and salt concentrations.

Incubation time is dependent on a number of factors including temperature, pH, ionic strength, etc. In general, binding of polypeptides can require as little as 5 minutes at 37°C up to 24 hours at 4°C. Similarly nucleic acid hybridization is dependent on the same physical conditions as well as being dependent on GC content of the nucleic acid. In general hybridization requires as little as 2 hours at 42°C and up to 48 hours at 42°C. Generally, hybridization is carried out at a temperature of about 20°C to about 50°C in about 0.15 M sodium chloride and 0.015 M sodium citrate for a period of about 1/2 hr. to about 18 hr. to allow formation of hybrids.

The techniques for the hybridization of DNA are disclosed in many references, including Walker and Gaastra (eds.) Techniques in Molecular Biology (1983) MacMillan Publishing Company, New York, pp 113-135 and 273-283; Maniatis *et al.,* (eds) Molecular Cloning (1982) Cold Spring Harbor Laboratory, pp 309; E. Southern, J.Mol.Biol. (1975) 98:503; Botchan *et al.,* Cell (1976) 9:269; Jeffreys et al., Cell (1977) 12:429.

The assay medium is preferably buffered at a pH in the range of about 6 to 9, with a convenient buffer such as phosphate, tris, or the like. The significant factor in selecting an appropriate buffer is that the buffer not inhibit the binding of the specific binding pairs.

The assay can be carried out at any suitable temperature which does not inhibit the desired reactions, generally about 20°C, but preferably at an elevated temperature below about 40°C. The assays are generally and preferably performed at atmospheric pressure.

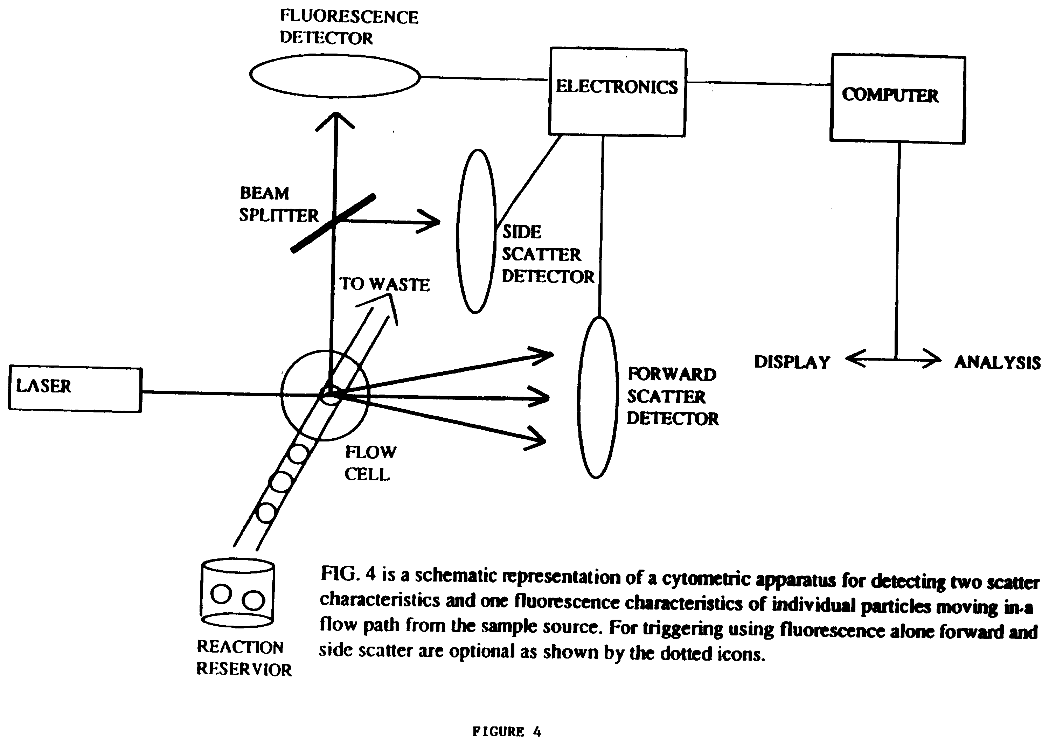

Following incubation, the labelled reagent is added and the incubation continued for 5 to 90 minutes. Preferably, standard solutions are prepared of known concentrations of analyte to serve as standards for comparison with the sample. In this way, accurate quantitative determinations can be obtained. Flow cytometry is the preferred method to detect the formation of, and optionally quantify, analyte-complementary binding moiety complexes and relate the information to the detection and determination of the amount of an analyte of interest present in the sample. The analysis of immunoreactive microspheres bound with a fluorescent dye in a flow cytometer can be determined using a flow cytometer of conventional design as represented in Figure 4 (See UK patent # 1561042). Alternatively, a modified flow cytometer may be used as described below.

Existing methods of performing contiguous analysis of multiple analytes rely upon the capability of a flow cytometer to simultaneously record side scatter and/or forward scatter of reflected light and light emitted from fluorochrome labelled microspheres. The intensity of forward scatter and side scatter of reflected laser light is determined by the size of the particle in the light path. The intensity of fluorescent light emitted at right angles to the light path is a function of the quantity of fluorochrome on the microsphere. The flow cytometer incorporates fluidics which permit only one microsphere at a time to cross the light path. Thus the size of the particle and its coincident fluorescence are co-determined.

In the convention of flow cytometers, flow microsphere-based immunoassays (FMIA) are performed in an appropriately configured reaction vessel. The reacted microspheres are passed through a flow cell which causes the microspheres to pass through a laser beam one at a time. The forward scattered light and side scattered light are gathered onto two detectors. The intensity of forward and side scattered light which is determined by the size of the particle in the beam is recorded with the appropriate electronics and stored in a computer for further analysis. The laser also excites the fluorescent dye specifically bound to the microsphere. The emitted light is collated to one of up to three fluorescence detectors. The intensity of the fluorescence is recorded with the appropriate electronics and stored for future analysis.

In this manner both the size and fluorescence of the microspheres are recorded. Since the intensity of fluorescence is determined by the number of fluorescent dye molecules associated with the microsphere a solid phase immunoassay can be performed on the microspheres and analyzed in the flow cytometer (Fulwyler and McHugh, Methods in Cell Biology, Academic Press 33, 1990). Forward scatter and side scatter or forward scatter alone has been employed to discriminate microsphere sizes. Forward scatter alone has been shown to discriminate particles that differ by less than 1 micron (McHugh in Immunochemica Vol 5, 1991). Previously all FMIA have been performed using fluorescence detection in conjunction either with both forward scatter and side scatter or with forward scatter alone.

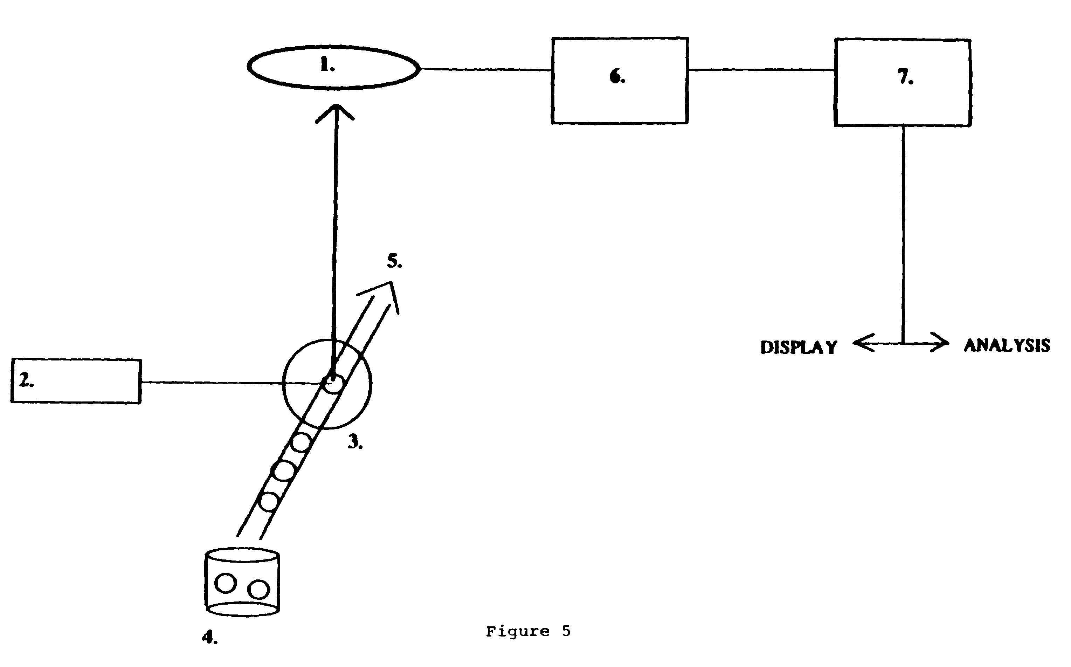

In using the present invention a flow cytometer of a specific design as illustrated in Figure 5 can be used instead of a traditional flow cytometer since there is no need to discriminate based upon microsphere size. So long as no size discrimination is needed, only fluorescence need be measured. In using the present invention, to perform analysis wherein it is necessary to discriminate microsphere size populations the apparatus shown in Figure 5 is modified to include a side scatter detector and the appropriate electronics as illustrated in Figure 4. With reference to Figure 5, the flow cytometer is as follows: FMIAs (or any other specific binding assay such as a DNA Probe-nucleic acid assay) are performed in a suitable reaction vessel (4), reacted microspheres (8) are passed through a flow cell (3) and discarded to a waste vessel (5), and a laser (2) is employed to excite the chromophobe used in the assay. (A laser is unnecessary if chemiluminescence or phosphorescence is utilized.)

The reaction vessel can be a test tube, a well of a microtiter plate or other container. The laser can be any of an Argon laser, a HeNe laser, a diode laser, a UV laser, an Arc laser, a krypton laser or any other light source which emits a light of wavelength suitable to excite the particular dye employed in the assay. The fluorescence emitted by the excited chromosphere is collated onto one or more fluorescence detectors (1). The occurrence of a fluorescent event of intensity at or above a preselected intensity is recorded in the appropriate electronics (6) for analysis as is the quantitation of that intensity. Data analysis is performed in a computer (7) using software of useful logic, or is performed manually.

As an internal control, included in the reagent mix can be a subpopulation of microspheres coated with the first of a binding pair wherein the second member of the binding pair is ubiquitous in samples to be analyzed by this method. Thus the absence of a positive resultant indicates that the method was employed erroneously thus serving as a control on the reproducibility of the method when, for example large numbers of samples are analyzed manually or by an automated method. Examples of such ubiquitous analytes include but are not limited to immunoglobulins, highly conserved genomes, glycoproteinaceous enzymes, glycogen, components of cell membranes and the like.

Where multiple subpopulations of binding moieties are combined in an appropriate assay medium with the addition of a control sample (no analytes are present) and labelled reagent, where the label is a fluorochrome, following excitation of the sample, a background measurement of fluorescent activity maybe obtained. For the most part, a low level of fluorescence will be obtained due to non-specific binding of the labelled reagent to the solid supports. This background fluorescence is not only acceptable, but is preferable in that it provides a means for identifying the subpopulation analytes which are absent from the sample. However, an essential requirement of this background activity is that it be distinguishable from activity which results from the presence of an analyte of interest in the sample.

The present invention contemplates assays in which the intensity of fluorescence decreases with the presence of analyte in the sample as is the case with competitive immunoassays, or fluorescence quenching assays of all types. In these cases both the histogram peak area and the mean channel fluorescence of the peaks associated with negative samples are predetermined by the reagent composition so that the amount of analyte can be calculated, i.e., the decrease in mean channel fluorescence of a peak of predetermined area is a direct measure of the amount of analyte in the sample associated with that peak area.

The present invention uses computer software to analyze data. The software logic interrogates histograms of number of events versus fluorescence intensity to determine the best fit of the data with all possible predetermined combinations. The software also traces the pattern of the peaks found in the histogram and identifies the peaks, valleys and shoulders so that the areas (i.e., number of events) of fluorescence intensity xl through x2, of each peak or combination of peaks can be calculated.

The analysis of the data in the fluorescence histogram is performed in a step wise manner as outlined below.
1. The histogram is made plotting the fluorescence intensity of the events acquired from the flow cytometer. See step 1.
2. The histogram is smoothed up to ten times using a 5 point Gaussian smooth. See step 2.
3. Significant features of the topography are identified. Valleys, peaks, the beginning and ends of plains are located on the smoothed histogram. (See step 3) The lowest bands represent valleys, the next highest bands represent peaks, the next highest bands represent the beginning of a plain, and the highest bands represent the end of a plain. A region simultaneously can be a valley and the beginning or end of a plain.
4. The regions identified in step 3 are used to define markers that separate the peaks. A valley is always assigned a marker. The beginning of a plain followed by the end of a plain is assigned a marker mid-way between the two regions. Other rules are used to assign markers when a region is simultaneously a valley and a beginning or end of plain. See step 4.
5. The markers may be adjusted if needed. If a region between markers has less than a certain number of events, it is joined to a neighbor. If there are more peaks than assays, markers are eliminated in a manner to get the best agreement between known microsphere proportions and observed proportions. If two peaks severely overlap, a peak overlap procedure calculates the proportions in the two peaks to improve accuracy. (See step 5)
6. The peaks are then assigned to the corresponding assay based on the area of the peak and the known proportions of the reagent. Regions between markers (peaks) are assigned to the assays based on the best fit between the observed proportions of the peak areas and the expected microsphere proportions. Every possible assignment of assays to peaks is evaluated, and the sum of expected proportion is determined. The best assignment is the one that gives the lowest sum of absolute differences. The mean channel fluorescence value is reported for each analyte as shown in Step 6.

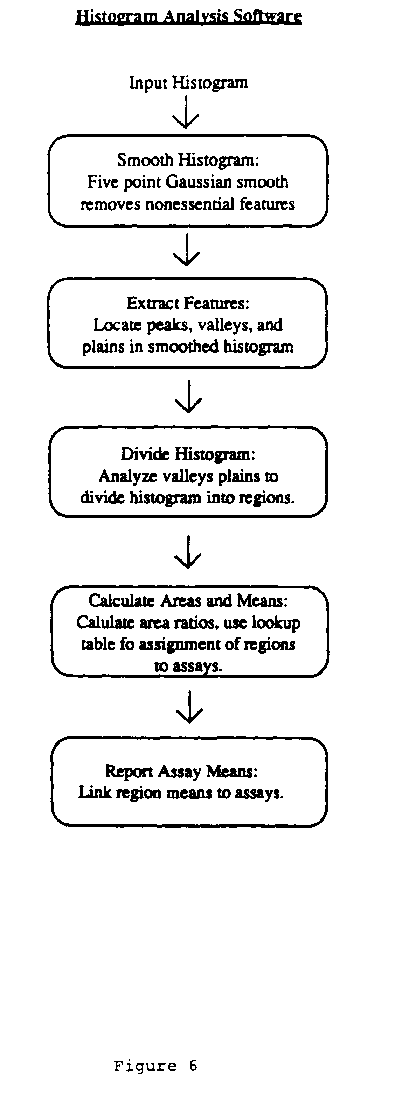

A flow chart outlining the major operations of the software is shown in Figure 6. Further, the source code of the software is attached as Appendix A.

Alternatively the data can be analyzed manually using printed histograms, such as those generated by commercially available flow cytometers and particle counters.

The present invention also contemplates a kit containing reagents for carrying out the present inventive method. The kit comprises containers of multiple subpopulations of complementary binding moieties to each of multiple analytes of interest generally each bound to populations a solid supports, wherein each analyte and its complementary binding moiety comprise first and second members of a specific binding pair (msbp). When bound to a solid support, the binding moieties may be provided in individual preselected proportions or as a mixture of preselected proportions. The kit may additionally include a separate container of a reagent labelled with a detectable label, particularly a fluorochrome which emits detectable fluorescence upon exposure to excitation energy, wherein said reagent is capable of binding to said first or said second msbp and allows for the detection of formation of any specific binding pairs.

Where the binding moieties are provided unattached to a solid support, the kit may additionally include solid supports and optionally reagents for attaching the binding moieties to the solid supports. Upon attachment of particular binding moieties to the solid supports of the kit, a mixture of the binding moieties can be formed in preselected unique proportions and the desired analyte of interest can be assayed.

The following examples are offered by way of illustration and not by way of limitation.

### EXAMPLES

### Experimental Procedures

### Materials:

Standard laboratory chemicals, purchased from various vendors including Sigma Chemical Co., Mallinckrodt, Inc., Fisher, Inc., etc., are used to make buffers.

Polystyrene microspheres of uniform diameters ranging from 2 to 10 microns (1 micron = 10⁻⁶m) can be purchased from several different vendors, including Polysciences, Inc., Seradyn, Inc. Bangs Laboratories, Inc., Flow Cytometry Standards Corp., and Duke Scientific, Inc.

Purified proteins, including conventional antigens as well as antibodies, to be coated onto the polystyrene microspheres may be derived from a variety of sources such as recombination DNA, tissue homogenates, viral, bacterial or cell lysates, and body fluids such as urine, plasma, ascites, etc. In many instances they can be purchased directly from vendors ("Linscott's Directory of Immunological and Biological Reagents", Sixth Edition, William D. Linscott, 1990-1991). If they are not readily available, they can be purified in the laboratory using conventional techniques. See Cuatrecausas *et al.*, "Selective Enzyme Purification by Affinity Chromatography, " Proc. Nat. Acad. Sci. 61:636-643 (1968); Goding, "Conjugation of Antibodies with Fluorochromes Modifications to the Standard Methods, " J. Immun. Meth. 13:215-226 (1976).

Fluorescein isothiocyanate (FITC) labelled affinity purified anti-IgG antibodies can be purchased from several vendors, including Caltag, Inc., Cappel, Inc., and Tago, Inc. Antibody purification and covalent coupling of FITC to purified IgG can also be accomplished in the laboratory using conventional techniques ("Methods in immunology and Immunochemistry", Eds., Curtis A. Williams and Merrill W. Chase, Volume 1, pp. 120-187, 1967, Academic Press. New York).

Polystyrene and polyethylene test tubes can be purchased from several vendors, including Baxter, Inc. VWR, Inc., and Fisher, Inc. Microtiter plates with or without filters, as well as vacuum filtration manifolds can be purchased from Millipore, Inc. and Pall Bio Corp.

### Reagent Preparation:

Preparation of the immunoreactive reagent involves two steps: (1) manufacturing immunoreactive beads (IRB) by coating polystyrene microspheres of a particular size with a protein antigen (Fulwyler and McHugh, In, "Methods in Cell Biology", Eds., H.A. Crissman and Z. Darzyneckiewicz, Vol. 33, pp. 613-619, 1990), and (2) mixing different preparations of IRB in predetermined proportions accurately. Other types of microspheres used includes polyacrylic microspheres, Montagne, *supra;* polyacrylamide and dextran microspheres, Lisi *et al., supra.*
(1) IRB Manufacture: A suspension of polystyrene microspheres is withdrawn out of the stock bottle, resuspended into fiftyfold excess volume of deionized water (dH₂O), washed by filtration and counted either by direct visualization in a microscope or electronically using a semi-automated particle counter. The microspheres are then resuspended in an appropriate buffer (e.g., 50 mM sodium borate, pH 9.5) and incubated with an appropriate concentration ratio of protein to microspheres. The incubation time and temperature as well as the concentration ratio of microspheres to protein are determined empirically. The microspheres are then separated from unreacted protein by filtration, washed and resuspended in a storage buffer.
   For example, IRB are employed for the detection of anti-HIV gp41 IgG antibodies in human sera. They are manufactured as follows: 4 micron microspheres (from Polysciences, Inc.) are washed as above and incubated with purified, recombinant DNA-derived gp41 (from DuPont, Inc.) in 50 mM sodium borate buffer, pH 9.5, at a concentration of 4 µg gp41/10⁷ microspheres/ml for 18 hours at 4°C. After washing and separation of unreacted gp41, the 4 micron-gp41 microspheres are stored at a concentration of 2 x 10⁷ microspheres/ml at 4°C.
(2) Preparation of a Proportional Reagent: The concentration of particles in each well-mixed stock suspension of microspheres attached to one member of a specific binding pair is measured by a particle counter, for example, a hemocytometer or a Coulter Multisizer II. The concentration may be measured directly, or indirectly by preparing and measuring the concentration of a known dilution of a stock suspension. An appropriate volume based on the desired concentration (proportion) of the specific IRB in the final reagent is then withdrawn from the stock suspension, and combined with other IRBs similarly aliquoted.

### IRB Assay:

The assay for detection of analytes in serum or any other medium typically follows a two-step protocol as described below. However, it should be noted that one-step assays are possible, and that the presently claimed invention is not limited to the following two-step protocol.
(1) Incubation of IRB with the medium to be screened: Sera or any other medium potentially containing the analytes of interest, with or without the sample preparation typical of binding moiety-type assays for the analytes, are mixed with IRB in an appropriate reaction buffer (e.g. PBS) at room temperature for 15 to 60 minutes. This may be carried out either in polypropylene test tubes or 96-well microliter plates with or without filters. Following the incubation period the microspheres are typically washed in order to separate unreacted analytes and minimize the binding of nonspecific components from the medium. The washing of microspheres can be accomplished either by centrifugation or by vacuum filtration if microliter plates with filters are used.
(2) Reaction with FITC-labelled Protein: If the analytes of interest are IgG antibodies in human serum, the microspheres from above are resuspended in buffer containing FITC-labelled antihuman IgG. If the analytes are other proteins in human serum or any other medium the FITC label used is a conjugate of FITC coupled to an IgG antibody that specifically reacts with the protein analyte or to a fluorochrome couple to another specific binding moiety which binds competitively to the coated microsphere or binds non-competitively to the analyte non-bound specifically to the coated microsphere. The microspheres are then incubated at room temperature for an additional 15 to 60 minutes, washed and resuspended in buffer for analysis in a flow cytometer of the type described in Figure 4.

### DATA ANALYSIS

Data analysis is performed in the following way using a flow cytometer such as the Beckton Dickinson FACScan:
1. Sample is introduced into the flow cytometer and an appropriate number of events (i.e. particles above the selected threshold value) counted, for example 4000 events counted. The number of events counted is determined by the number and proportion of discrete subpopulations with specific binding pairs which are contained within each assay. The events can be triggered by forward scatter, side scatter, or fluorescence above the appropriate threshold level. For example, a forward scatter threshold may be set at channel 348, on a scale of 0 to 1024 channels, to eliminate particles less than 2 microns in size, if desired.
2. The computer collects data on each particle for each of the preselected parameters such as forward scatter (FSC or FACS), side scatter (SSC), fluorescence 1 (FL1), etc.
3. The data is configured to output in the form of a fluorescence histogram where the Y-axis reports the number of events corresponding to each fluorescent channel shown on the X-axis. Depending on the design of the experiment one or more peaks will appear in the histogram.
4. Appropriate markers are set at florescent channel values to separate and calculate the area of each peak. The area of each peak as well as its mean channel fluorescence (MCF) is diagnostic of the sample being examined.

### EXAMPLE 1.

### STRICT CORRELATION OF REAGENT PROPORTIONS AND HISTOGRAM PEAK AREA.

A reagent was prepared using 4 micron diameter microspheres wherein the microspheres were coated with either recombinant HIV antigen gp41 or affinity purified goat antihuman IgG. Reagents containing exclusively either or both of these coated microspheres were blended proportionately so that microspheres coated with gp41 antigen comprised (within procedural error) 100%, 89%, 79%, 68%, 58%, 48%, 38%, 28%, 19%, 9% and 0% of the number of total microspheres in the reagent and microspheres coated with IgG antibodies comprised (within procedural error) 0%, 11 %, 21%, 32%, 42%, 52%, 62%, 72%, 81%, 91% and 100% respectively. Each of these 11 proportional reagents were contacted with human serum known to be positive for both IgG and gp41 antibodies by Elisa techniques and incubated for 40 minutes at room temperature. After washing a second reagent containing fluorescein(FITC) conjugated antihuman IgG antibodies (0.0076 mg/ml, molar ratio F:P 5) was added to the reaction vessel and incubated for 20 minutes at room temperature. After washing the quantity of FITC associated with the reacted microspheres was analyzed by delivering the reaction mixture into a Becton Dickenson FACScan flow cytometer of a type as illustrated in Figure 4 wherein 4000 events were accumulated by triggering on forward scatter and the fluorescence intensity of each event was recorded in computer memory.

These data were analyzed and output as a histogram of number of events (Y axis) versus log of fluorescence intensity (X-axis). 4000 total events were acquired via forward scatter triggering. Under these conditions one or two histogram peaks was observed depending upon whether the reagent contained one or two types of microspheres respectively. Them peaks were observed as expected for a serum positive for both IgG and gp41 antibodies, i.e., two nonoverlapping peaks. The areas of the peaks were calculated manually by placing a marker between the peaks and noting the number of events on either side of the marker. The peaks were observed to have different areas and the mean channel fluorescence of the peaks was consistent. The magnitudes of the peak areas thus measured varied in a manner predicted by the proportionality of microspheres in the reagents within experimental error as follows wherein the peak area was expressed as number of events within the peak as a percentage of the total number of events acquired: peak A contained 100.0%, 78.4%, 76.9 %, 62.1%, 49.3 %, 39.3%, 31.5%, 25.7 %, 19.7%, 11.0% and 0.8% respectively of the total events acquired which percentages correlate with the reagent microsphere percentages first listed above for gp41 coated microspheres with a coefficient of correlation of 99.2% and peak B contained 0%, 17.0%, 28.0%, 33.7%, 45.6%, 50.8%, 60.6%, 66.5 %, 72.5 %, 81.6%, and 90.5% respectively which percentages correlate with the reagent microsphere percentages secondly listed above for IgG coated microspheres with a coefficient of correlation of 99.8%. Variance can be accounted for by error in the counting, measuring and mixing of coated microsphere reagents.

### EXAMPLE 2.

### HISTOGRAM AREA IS INDEPENDENT OF FLUORESCENCE INTENSITY.

Using the methods described above it has been observed that the fluorescence intensity of a positive peak in a histogram can vary from one positive serum to another. The fluorescence intensity is reputed to be a function of the antibody titer. A serum known to be positive for HIV gp41 antibody was serially diluted lx, 2x, 4x, 8x and 64x with a known negative serum. These panels of diluted serum were separately contacted with one of four reagents containing, respectively, 80.7%, 61.1%, 41.1% and 20.7% of 4 micron microspheres coated with gp41 antigen. In these experiments a histogram peak was observed which moved to the left on the X-axis as dilutions were increased such that the fluorescence intensity decreased from 890 to 763 to 613 to 458 to 102 respectively with the dilution factors listed above. Irrespective of the fluorescence intensity of these families of peaks, the peak areas expressed as a % of the total events acquired was observed to be 75.1%, 57.7%, 40.5 %, and 22.1 % with respect to the gp41 coated microsphere proportions listed above.

### EXAMPLE 3.

1. THE SUMMED AREA OF OVERLAPPING PEAKS IS DETERMINED BY THE PROPORTIONALITY OF THE REAGENT MICROSPHERES WHOSE FLUORESCENCE CONTRIBUTES TO THE COMBINED PEAK IN THE HISTOGRAM.
2. THE AREA OF A HISTOGRAM PEAK ARISING FROM NONSPECIFIC BINDING, I.E. A NEGATIVE, IS DETERMINED BY THE PROPORTIONALITY OF THE RESPECTIVE MICROSPHERES IN THE REAGENT.

Using the methods described above, it was observed that the histogram peaks of two sera each positive for a different antibody can exhibit the same or nearly the same fluorescence intensity value. Thus it is possible that a serum positive for two analytes might exhibit one peak, i.e. an overlapping peak or partially overlapping peaks. Proportional microsphere reagents were prepared which contained 4 micron diameter microspheres coated with IgG antibodies or HIV antigen gp24. A first set of reagents was prepared which contained 20% of p24 coated microspheres in which the proportion of either gp41 or IgG antibody coated microspheres was 80% (within procedural error). These two reagents were each contacted with a serum positive for gp41 antibodies and IgG but negative for p24 antibodies. In this first instance two peaks were observed in each of the two histograms, one in the left hand region (mcf 372) of the histograph of area 23% indicating a negative p24 antibody resultant and one further to the right. The right most peaks in these cases each had an area of 76% of total counted events and both exhibited mean channel fluorescence values of 624 and 628 respectively indicating the potential for overlapping peaks had both gp41 and IgG antibody coated microspheres been included in the reagent.

A second set of reagents was prepared wherein the proportion of p24 microspheres was 17.7% of the total (within procedural error). In this reagent set microspheres coated with gp41 or IgG antibodies were both added to comprise 82.3% of the total number of microspheres wherein each was added reciprocally in varying amounts from 0% to 82.3% to make a set of 5 reagents each with a different IgG and gp41 antibody proportion. These reagents were contacted with the serum above which was p24 antibody negative, IgG and gp41 antibody positive and in which the IgG and gp41 derived peaks were expected to overlap. The observed patterns in each of these five cases revealed a left most peak of mean mcf value 366 and an average area of 23.6 % of the total events indicative of the p24 antibody negative status of the serum. A second, right hand singlicate peak of mean mcf 621 was also observed in all 5 cases with an average area of 76.4% indicating a consistent sum for the combination of gp41 antibody and IgG irrespective of their individual microsphere proportions.

Another set of 5 three microsphere reagents were prepared wherein the proportion of p24 coated microspheres was 77.6% and the gp41 and IgG antibody coated microspheres were added in varying and reciprocal amounts to total 22.4% (on average, within procedural error). When these 5 reagents where separately contacted with the same serum as above a pattern like that reported above was observed except that the left hand peak held an area of 79.5 % of the total events and the right hand or gp41 antibodies and IgG positive peak had an average area of 20.5 % of the total events.

### EXAMPLE 4.

### UNIQUE MICROSPHERE REAGENT PROPORTIONS

It is known that serum can be positive for many different antibodies as for example in a blood bank infectious disease screen. Since the MCF of a positive histogram peak may shift with the particular antibody titer it is possible that many positive peaks could overlap when using an immunoreactive microsphere assay as described here. To perform a discrete analysis proportional reagents are prepared in such a way as to cause every possible combination of overlapping histogram peaks to have a unique area.

The coated microsphere subpopulations are each prepared separately, counted, and then combined into a multiple subpopulation reagent in which the relative proportions of each subpopulation by number of microspheres is by design unique. If a 3 part reagent were prepared in which the subpopulation proportions were 1:2:3 (16.67%/: 33.33 % : 50%), and then was reacted with an unknown sample which was positive for the first two of the three target analytes as listed an indeterminant result would be possible. If the mcf values of the positive peaks were by coincidence the same or nearly so, i.e., overlapping peaks, the histogram would show two peaks of equal size each comprising 50% of the total number of events analyzed. One of these peaks would be in the left hand region, or negative domain, of the histogram, and one would be in the right hand positive region. From this resulting pattern, it would not be possible to discern whether the sample was positive for both the first and second analytes as listed or positive for the third analyte only. However, if the reagent had been designed with proportions 1:2:4 (14.28%, 28.57%, 57.14%), the resulting histogram would be uniquely interpretable. A sample positive for the first two analytes as listed wherein their peaks overlapped would show a right hand or positive peak of area 42.85% and a left hand or negative peak of 57.14%, thus correctly identifying the respective analytes unambiguously since 42.85% could only be the sum of 14;28% and 28.57 %. This example demonstrates the necessity and utility of the design of combination reagents of unique proportions.

The essential property of such proportions is that each proportion or combination of proportions is unique from every other possible proportion or combination of proportions. A reagent containing two subpopulations of detecters combined in the proportions 1:2 will exhibit that property, as will a 3 part reagent of proportions 1:2:4, as will a 4 part reagent of proportions 1:2:4:8. In the general case, a multiple subpopulation reagent in which the numbers of microspheres in each subpopulation added is in turn twice that of the successive subpopulation will exhibit the desired property of unique summing. The algorithm **n: 2n: 4n: 8n . . . :2**^{**(m1)**}**n** can be utilized to determine the proportions of unequivocal analytical utility to be used in the preparation of a reagent containing m subpopulations of coated microspheres of similar diameter, and wherein n is any number.

In cases where the number of reagents is small, proportions other than those in the doubling algorithm above may suffice. For example, in the case of a two part reagent, any ratio of microsphere subpopulation other than 1:1 can be useful, such as 1:3 or 1:5, since there is only one arithmetic sum possible. Similarly, in the case of a three part reagent, other proportions will work, such as 1:3:5 or 1:9:15. As the number of subpopulations increases, however, the number of distinct algorithms which exhibit the property of unique nonsumming proportions decreases. The choice of which of the potential algorithms to utilize in the design and preparation of the multiple subpopulation reagent is a practical matter which is influenced by the particular requirements of the assay in question.

The logic of unique microsphere subpopulation proportions discussed above applies to m subpopulations of microspheres of the same mean diameter. Thus, a number of discrete contiguous assays are possible wherein only microspheres of one size are employed.

In addition, it is possible to prepare reagents of this general type using two or more populations of microspheres of differing mean diameter. In this case, the unique summing algorithms will apply to each of the differing microsphere size populations independently of the other. For example, a 4 micron diameter microsphere three part reagent of proportions 1:3:5 could be combined with a 10 micron diameter microsphere four part reagent with proportions 1:2:4:8 to comprise a seven part reagent for the contiguous analysis of seven different analytes. If the flow cytometer is properly tuned to analyze the 4 micron diameter microspheres and the 10 micron diameter microspheres in different gates, the properties of unique proportions and arithmetic sums of overlapping peaks will hold in each gate.

### EXAMPLE 5.

### NON-UNIQUE REAGENT PROPORTIONS

Reagents are devised wherein the proportions of microspheres are not uniquely determined such that nondiscrete analysis is performed wherein the presence of one or more of a class of analytes is determined but the particular analyte cannot be identified. Reagents are also configured to perform discrete analysis of some analytes and nondiscrete analysis of others. Specifically, a reagent is made with microspheres of a single size containing subpopulations of these microspheres differently coated. Each of the subpopulations of microspheres are added together in equal amounts to prepare a reagent of proportion 1:1:1:1.

When such a reagent of non-unique proportions is contacted with a sample which is positive for one or more of the targeted analytes, one or more peaks will appear in the right hand or positive side of the histogram. Several possible histogram patterns can emerge from such a situation depending on how many of the targeted analytes are present in the sample and the mcf value of each resulting peak. In none of the cases will the presence of a single noncompound peak indicate which analyte is present because the peak areas of each distinct analyte are the same. For example, for a 4 part non-unique reagent (1:1:1:1), each peak will have area of 25% of the total counted events. In the situation where overlapping peaks occur, and the areas sum up to 50%, 75%, etc., it is possible to say whether one, two or three of the targeted analytes is present but not which one, two or three. Thus the numbers and areas of the resulting peaks on the right hand side of the histogram can be used to determine the presence or absence of target analytes in general, and the number of analytes present. Only in the case where 100% of the targeted analyte types are present is it possible to unequivocally identify the targeted and positive analytes because all are present, i.e., 100%/ of the histogram area is on the right hand side of the histogram, either as distinct peaks or overlapping peaks.

Non-unique proportion reagents as thus described may be employed usefully as screening assays where the identification of the particular analyte may be less significant than the simple identification of a positive sample, a class-positive screen. This case does not necessitate achieving any particular accuracy in the preparation of a 1:1:1:1 reagent. For example, 1:1.2:1.1:1.25 proportions may suffice. Similarly, it is sufficient that the numbers of microspheres in each subpopulation are similar even though not known with any accuracy. With a reagent of this type, only a simple group positive result is possible. The determination of how many analytes were positive by investigation of the areas of the peaks would be impossible except where 100% of the histogram area is on the right hand side.

Microspheres which have been contacted with a sample positive for the analyte to be detected by that specific microsphere have a higher MCF value than analyte-negative samples. Detection of peak in the positive region of the histogram indicates presence of some positive analyte. A larger peak in the positive region indicates a larger number of analytes.

A reagent was prepared which contained substantially equal proportions of 4 micron diameter microspheres coated with IgG antibodies, recombinant HIV p24 antigen (p24), recombinant HIV antigen gp41 (gp41), or HCV-333 (HCV) antigen coupled to bovine serum albumin.

This reagent was contacted with sera positive for various analytes as indicated in the following table, followed by staining with FITC-aIgG. Results were analyzed by placing a cutoff marker at the 550 channel of fluorescence, peaks which yielded mean channel fluorescence (MCF) values below 550 were considered negative; peaks which yielded MCF values above 550 were considered positive.

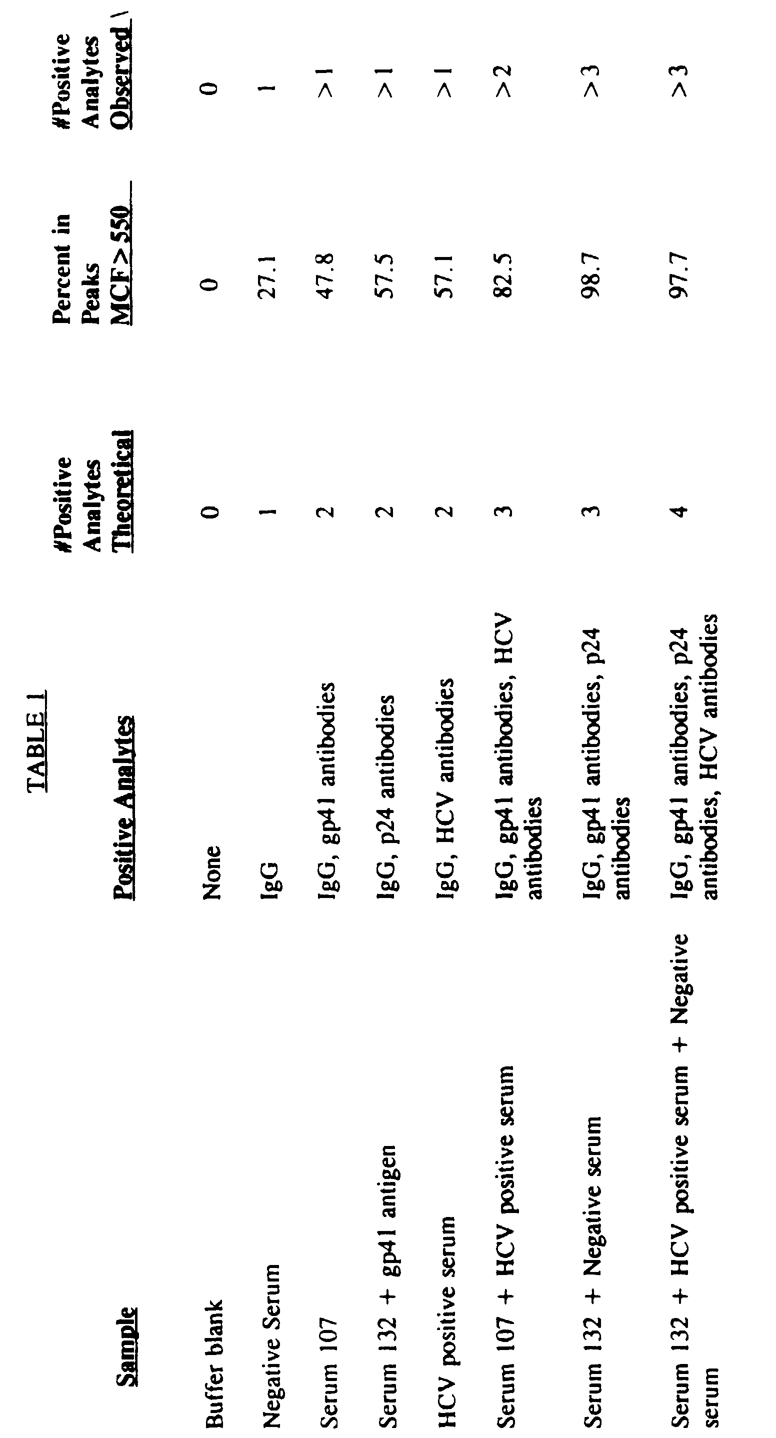

### EXAMPLE 6.

### HETEROGENOUS MICROSPHERE SIZE REAGENTS

Proportional reagents are prepared wherein not all the microspheres are the same size. The forward scatter trigger is set so as to include as counted events all the different size microspheres employed. Such a heterogeneous proportional reagent exhibits the same features as have been described in examples one through five above.

For example, a reagent could be prepared which contained 2 micron, 4 micron and 10 micron diameter microspheres, with each population differently coated to create a 3 analyte multiplex immunoassay and combined in the proportions 1:2:4. If the reacted reagent were analyzed in an ungated flow cytometer or gated so that all microsphere events of diameter equal to or greater than 2 microns were stored in the same histogram, the peak areas would exhibit the predicted relative values, i.e., 14.3%, 28.6% and 57.1%.

### EXAMPLE 7.

### GATED MULTIPLEX REAGENT ANALYSIS

Proportional reagents are prepared wherein microspheres of two or more size classes are mixed. Each microsphere size class is coated as described above and blended in predetermined proportions. The coated microsphere size classes each proportionately blended are then added together to form a reagent containing all the various microsphere size subpopulations. The assay and analysis is performed as previously described in an apparatus of the type shown in Figure 4 except that the histogram for each microsphere size subclass is analyzed using the "gating" technique described by Fulwyler, McHugh and others. Each histogram when analyzed individually exhibits the unique peak area distributions as have been described in example one through five above. Accordingly a number of discrete assays can be performed on one microsphere size contiguously with a second number of assays performed on a second microsphere size and so on.

For example, a five part immunoassay was configured in which a 3 part 4 micron diameter microsphere reagent in the proportions 5:7:14 was combined with a 2 part 10 micron diameter microsphere reagent in the proportions 3:4. specifically, the 4 micron diameter microspheres were coated with either HCV antigen, HTLV antigen or HIV gp41 antigen, and these microsphere preparations were combined to yield a reagent with 19.2 %, 27.8% and 53% of these subpopulations, respectively. The 10 micron diameter microspheres were coated with IgG antibodies or HIV p24 antigen and combined to yield 42.7 % and 57.3%, respectively.

This blended reagent then was exposed to various sera positive for one or more of the target analytes. Five microliters of serum was contacted with 200 microliters of reagent containing approximately 200,000 total microspheres: 140,000 4 micron diameter microspheres and 60,000 10 micron diameter microspheres. The first reaction proceeded for 40 minutes at ambient temperature in a microtiter well. After the first incubation, the well was washed with buffer and rinsed by vacuum several times. A second reagent comprised of FITC labelled goat antihuman IgG antibody was added for 20 minutes at ambient temperature and rinsed as before. The microspheres were resuspended in 200 microliters and injected into a FACScan flow cytometer. The cytometer was set to trigger on forward scatter so that the histograms of the 4 micron diameter microspheres and the 10 micron diameter microspheres passing through the flow cell could be analyzed separately. 10,000 total events were recorded.

For example, the results of the analysis of a serum positive for IgG, HIV gp41 antibody and HIV p24 antibody, but negative for HTLV antibody and HCV antibody were as follows. The histogram representative of the 4 micron gate included 6597 total events and revealed 3 peaks. These peaks had mcf values of 290, 419, and 709, and areas of 1355, 1626, and 3616 events, respectively. The histogram representative of the 10 micron gate included 2504 total events and revealed one peak. This peak had an mcf value of 783 and an area of 2504 events. In the four micron gate, the three peaks in order of increasing mcf (290, 419, 709) had relative areas (peak area in events/total histogram area) of 20.5 % (1355/6597), 24.6% (1626/6597), and 54.8% (3616/6597), respectively. These relative areas correlate with the 4 micron diameter subpopulation proportions of HCV, HTLV, and HIV gp41, respectively (19%, 27% and 53%), and the identification was thus made. A mcf value for HCV antibody of 290 and a mcf value of 419 for HTLV antibody were both below the previously determined cut-off value and so the sample was given a correct negative assignment for these two analytes. The third and largest peak, identified as HIV gp41 with an mcf of 709 was well above the cut-off mcf for this antibody, and thus the sample was deemed positive for HIV gp41 antibody.

In the 10 micron gate, a single peak of relative area, 100%, with a high mcf of 783 reveals the positive presence of both IgG and HIV p24 antibody. The peak is a compound peak comprised of both subpopulations of microspheres in the 10 micron diameter reagent.

By using the two microsphere size, five part reagent described, and with a gated analysis, the sample was correctly identified as IgG positive, HIV gp41 antibody positive, HIV p24 antibody positive, HCV antibody negative, and HTLV antibody negative.

In a second example, the results of the analysis of serum positive for IgG and HCV but negative for HIV gp41, HIV p24 and HTLV were as follows: The histogram representative of the 4 micron gate included 7451 total events and revealed 3 peaks. These peaks had mcfs of 287, 587, and 797, and areas of 3723, 2191 and 1537 events, respectively. The histogram representative of the 10 micron diameter microspheres included 1894 total events and revealed 2 peaks. These peaks had mcfs of 592 and 762 and areas of 1040 and 854 events respectively.

In the 4 micron gate, the three peaks in order of increasing mcf (287, 587 and 797) had relative areas of 50%, 29.4 % and 20.6%, respectively. These relative areas correlate with the 4 micron subpopulation proportions of HIV gp41, HTLV and HCV in that order (53%, 27.8% and 19.2% in the designed reagent). Only the HCV peak (the smallest one) in the 4 micron gate had a mcf value sufficiently high (797) to give the sample a positive assignment. In the 10 micron gate, the two peaks of mcf values 592 and 762 had relative areas of 55% and 45%, respectively. which correlated with HIV gp24 and IgG in that order. From a previous determination of the requisite cut-off value, a mcf of 592 is not sufficient to assign a positive HIV gp24 antibody to this sample. Accordingly, the sample was identified as IgG positive. By this method, the serum sample correctly was assigned as HCV antibody positive, IgG positive, and negative for all of HIV p24 and HIV gp41 antibodies.

### EXAMPLE 8.

### FLUORESCENCE TRIGGERING

In the previous examples the functionality of forward scatter in an apparatus as depicted in Figure 4 was utilized to acquire a preselected number of events into the fluorescence intensity data set. In those cases a trigger or threshold was set to select as countable events only those microspheres passing through the flowcell of a certain minimum size, for example 2 microns diameter. An advantageous analysis was done whereby forward scatter was not utilized as the event trigger but fluorescence intensity above a certain preselected value was used to acquire events into the data set. One advantage of this method of analysis was that none of the functions, optics or electronics of forward scatter or side scatter was necessary.

An experiment of design as in example one above was performed wherein 4 micron diameter microspheres were counted with either HIV antigen gp41 or IgG antibodies. Proportional reagents were prepared in which the gp41 coated microspheres comprised 100%, 89%, 79%, 69%, 58%, 48%, 38%, 29%, 19%, 9% and 0% of the total microspheres respectively and the IgG antibody coated microspheres made up the balance in the reagents. These reagents were contacted with serum known to be positive for both IgG and gp41 antibody. The completed reaction mixture was delivered to a FACScan flow cytometer (Becton Dickenson) of design as depicted in Figure 4. The analysis was performed in three ways: 1) triggering was set on forward scatter at a threshold value of 348 to exclude particles of less than 2 micron from acquisition into the data set, and 2) triggering was set on fluorescence intensity at FL1 settings of 400 or 450 to exclude from acquisition into the data set varying degrees of the low intensity fluorescence noise coming from fluorescent debris of ubiquitous origins.

Inspection of the histograms derived from each of the experiments above revealed two peaks (except in the case of 0% microsphere proportion): one of average mcf of 517 and a second of average mcf 725 indicating the positive presence in the serum of both IgG and gp41 antibody. These peaks were non overlapping in all cases. A marker was set between the two peaks by inspection of the first histograph printed out and this setting was used throughout the rest of the histograms. The total number of events on each side of the marker, i.e. the area of each peak, was recorded and was enumerated as a percent of the total events acquired.

The peak areas of the rightmost peak (gp41, deduced as follows by the coincidence of reagent proportion and peak area) in the forward scatter triggered evaluations were 89%, 80%, 70%, 60%, 50%, 43%, 33%, 17%, 8%, and 0%. The coefficient of correlation (r squared) of these data with the respective gp41 coated microsphere proportions listed above was 0.9928. The peak areas of the rightmost peaks in the fluorescent trigger of 400 experiments were 87%, 77%, 68%, 62%, 54%, 45%, 34%, 26%, 17%, 9.5%, and 3% to yield a coefficient of correlation of 0.9982 with the microsphere reagent proportions as above. Fluorescence triggering at a threshold of 450 yielded nearly identical results with a 0.9952 correlation coefficient. A similar numerical analysis for the left most peak in the histograms (IgG by deduction) yielded the expected reciprocal numbers and high coefficients of correlation. Analysis of covariability indicated that much of the lack of absolute correlation could be accounted for by subjective error in the placement of the marker separating the peaks.

These results indicate that the tautology of preselected proportionality of reagent microspheres coated with a specific member of a binding pair with the area of the resultant histograph peak holds whether forward scatter (i.e., microsphere size discrimination) triggering or fluorescence intensity triggering is employed to define the total number and type of events collected into the data set. Thus it is not necessary for purposes of these analyses to employ or even have present in the flow cytometer the devices (detectors, lenses, and electronics) required for other applications of the flow cytometer. Thus an apparatus devoid of these devices as depicted in Figure 5 could be employed with good result.

### EXAMPLE 9.

### A FOUR ANALYTE HIV ASSAY

Using the methods described above and utilizing forward scatter as a trigger, an analysis of known positive serum was performed using 4 micron diameter polystyrene microspheres coated with IgG antibodies and antigens HIV gp41, HIV p24, and hepatitis B core protein (HBc) in the proportions 13%, 53%, 27% and 7% respectively. The resultant histogram of a known serum positive for all four analytes had four peaks, each of unique size as follows wherein 5000 total events were plotted: 663 events (13% mcf 640), 2522 events (50% mcf 731), 1406 events (28% mcf 822), and 324 events (6.5% mcf 421) respectively. Other sera which were negative or positive for one or more of the analytes each exhibited unique peak patterns which allowed the correct qualitative interpretation of the serum reactivity.

A multiplex regent was prepared using 5 subpopulations of 4 micron mean diameter microspheres blended in the proportions 1:2:4:8:16. Microspheres coated with IgG antibodies comprised 3.2% of the final reagent. Microspheres coated with a recombinant HCV antigen comprised 6.5%, microspheres coated with RE-5 HTLV antigen comprised 12.9%, microspheres coated with HIV p24 antigen comprised 25.8 % and microspheres coated with HIV gp41 antigen comprised 51.6% of the blended reagent, respectively. A look-up table was prepared which listed all the possible unique combinations of peak areas. For example, a compound peak comprised of reacted microspheres for HCV, HTLV and HIV gp41 antibodies would be predicted to have a relative area of 45.2 %, and this value was recorded on the table along with all other possible combinations.

This reagent was reacted with a number of sera under standard protocols, 20,000 total events were recorded using a forward scatter trigger, and results were tabulated via the manual method. Some of the sera tested were blends of several known positive sera to create a number of multiple positives. Two typical analyses described below serve as examples.

In the first example, the histogram contained four peaks: the largest peak of relative area, 77.38%, and mcf of 302; the smallest peak of relative area, 3.95%, and mcf of 605; a third peak of area, 11.29%, and mcf of 696; and a fourth peak of relative area, 7.33%, and mcf of 776. By reference to the look-up table, the first and largest peak was identified as a double peak comprised of HIV gp41 and HIV p24. Since the mcf of this peak was below the previously established cut-off values, this sample was identified as negative for both HIV antibodies. The smallest peak was identified as IgG and the mcf was sufficiently large to identify the sample as IgG positive. The third and fourth peaks had relative areas of 11.29% and 7.33%, which associated them with HTLV and HCV, respectively. The mcf values of both these peaks was higher than the pre-established cut-off values for either HTLV or HCV antibodies, and thus the sample was identified as positive for both. By referring to the list of serum samples, this sample was determined to be a blend of two sera, one negative for all but IgG and the other positive for IgG, and HCV and HTLV antibodies.

In the second example, the resultant histogram had two peaks: a small peak of relative area, 5.1 %, and mcf of 314, and a second compound triplet peak of total relative area, 93.3%, and mcf of 744. By reference to the look-up table, the small peak was associated with a HCV negative identification. The total area of the larger peak was uniquely associated with the combination of IgG and HTLV, HIV p24 and HIV gp41 antibodies. Both the mean mcf and the distinct mcfs of each peak top were well above the pre-established cutoff values for IgG, HTLV antibody and the two HIV antibodies. Thus this sample correctly was identified as HCV negative, and IgG, HTLV, HIV gp41 and HIV p24 positive, despite the occurrence of only two clearly distinct peaks.

### EXAMPLE 10.

### Contiguous ANTIGEN AND ANTIBODY CAPTURE

It is known that antigens and antibodies of diagnostic interest occur in the same sample. Therefore it is of use to have a reagent which is able to contiguously identify both antigen and antibodies. This example also demonstrates that identification of analyte can be made when the relative placement of some of the positive and negative peaks were reversed.

A reagent was devised using 4-micron diameter microspheres wherein the microspheres coated with one of recombinant HIV gp41 (gp41), recombinant HIV p24, or mouse ascites fluid containing a mouse monoclonal antibody to the beta subunit of human chorionic gonadotropin (hCG) were combined such that the gp41-coated microspheres comprise 47.4 % of the total microspheres, the p24-coated microspheres comprised 16.7% of the total microspheres, and hCG-coated microspheres comprised 35.5% of the total microspheres. This reagent was contacted with various sera as indicated in the table below, followed by contact with the labeling reagent which FITC-antiIgG plus fluorescein-labeled human chorionic gonadotropin. Results in this example were consequently of two types, one type in which a negative sample exhibited a low MCF value and a positive sample exhibited a high MCF value; and a second type in which this was reversed. The first type was that exhibited by the p24-coated microspheres and the gp41-coated microspheres, and the second type was exhibited by the hCG-coated microspheres.

These theoretical values were based upon observation of single microsphere types (MCF values) and percent of microspheres in well-separated peaks:

| Analyte | Theoretical Percent in Peak | Avenge MCF of single microsphere type | |
|---|---|---|---|
| | | Negative sample | Positive sample |
| p24 | 16.8 | 497 | 816 |
| hCG | 35.5 | 483 | 336 |
| gp41 | 47.4 | 273 | 64 |

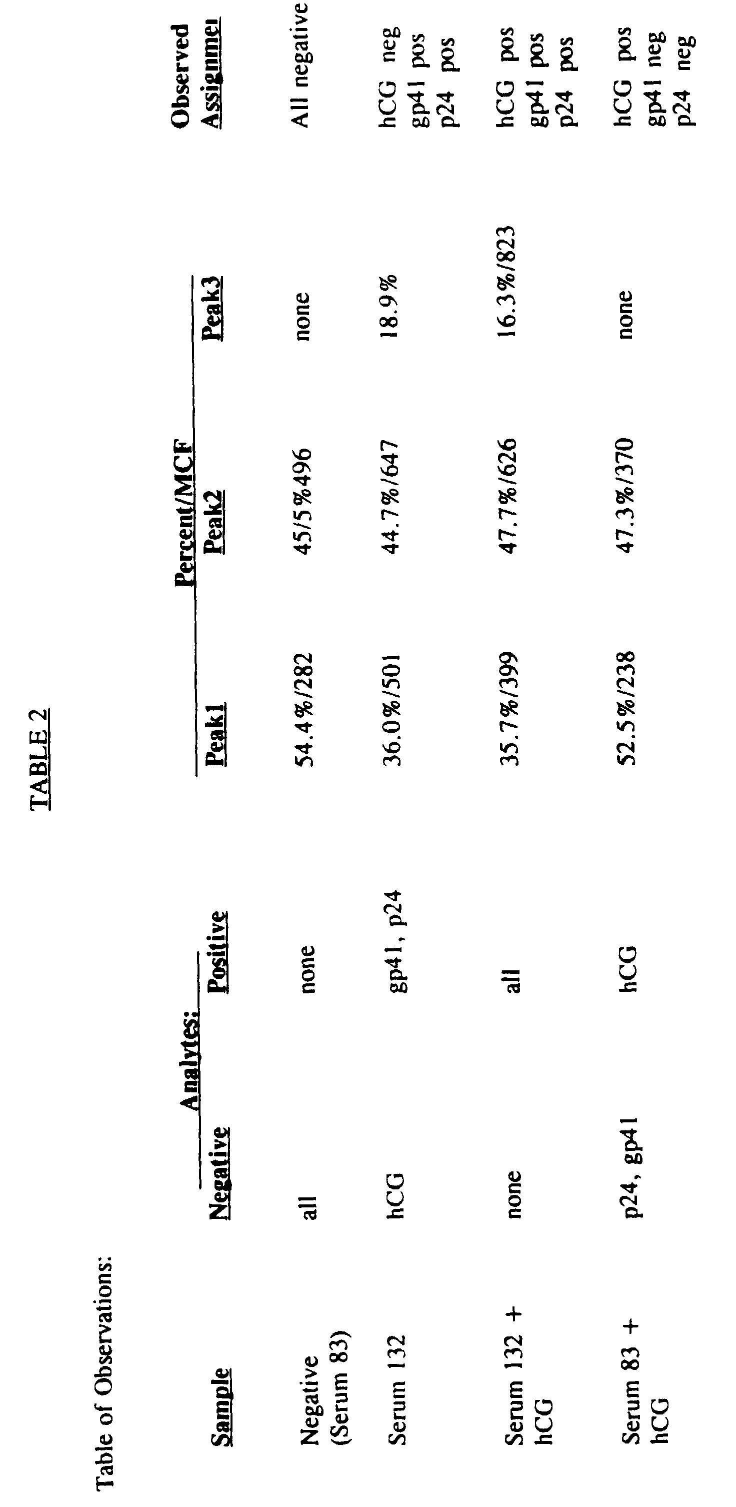

### EXAMPLE 11.

### QUANTITATION OF ANALYTE

A reagent was devised wherein all microspheres were of the same size, and the proportions were unique. Samples containing analytes were serially diluted into a sample not containing all analytes. The dilution factor was compared with the MCF values of the resulting peaks to demonstrate that differing concentrations of analyte in sample resulted in differing MCF values in an expected fashion.

A proportional reagent was prepared wherein 4-micron diameter microspheres coated with IgG, recombinant HIV p24 (p24), or recombinant HIV gp41 (gp4l) were combined in the proportions 13.2%, 24.9%, and 60.3%, respectively. The reagent was contacted with serum samples prepared by serial dilution of a gp41 positive, p24 positive, IgG positive sample into a gp41 negative, p24 negative, IgG positive sample, followed by contact with FITC-antiIgG. In the following table, peak 1 is the rightmost peak, peak 2 the middle peak, unless there are only two peaks in the instances of combined peaks, and peak 3 is the leftmost peak.

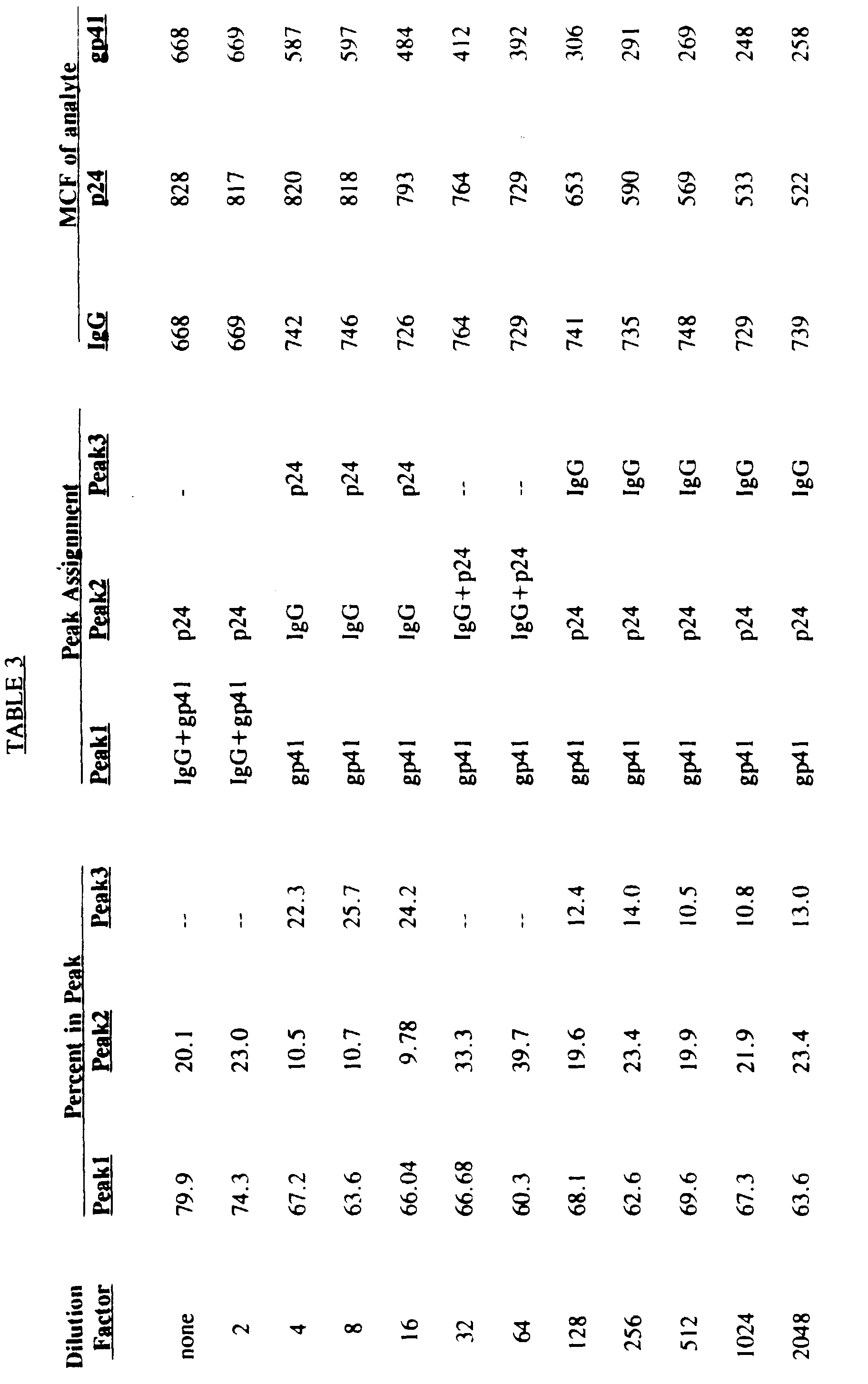

### EXAMPLE 12.

### METHODS OF DATA ANALYSIS

Histograms containing multiple peaks, some of which may be compound peaks, are interpreted for the information contained therein by either of two methods, a manual method or a computerized method using custom software.

In the manual approach, the histogram was displayed on the computer screen using software furnished by Becton Dickenson as a usual component of their flow cytometry data display products. By visual inspection of the histogram, display tabs were set on the X-axis on either side of each peak such that the entire histogram is divided into a number of domains each containing observable peaks or doublets, triplets, etc. In addition to printing the histogram, the computer will print out the total events in the entire histogram, the number of events between each pair of tabs and the percentage of the total histogram events included in the bordered domain of each tab. By comparing the relative peak areas thus determined with the known possible reagent subpopulation combinations, the unique identity of each peak or compound peak was ascertained.

In the computerized method of data analysis, the approach is the same as in the manual method except that a custom software program is used to set the tabs in the optimum locations via an iterative algorithm as follows:
1. input histogram
2. smooth histogram: a five point Gaussian smooth removes nonessential features
3. extract features: locate the peaks, valleys and plains in the smoothed histogram
4. divide histogram: locate valleys and plains to divide histogram into regions
5. edit peaks: suppress debris and aggregate peaks, suppress markers set excessively high above background
6. calculate areas and means of regions
7. assign analytes to designated regions: calculate area ratios, use look-up table for assignment of regions to assay analytes
8. report assay means: correlate mcf to assay analytes
9. output results

The present invention includes the capability to perform qualitative, semiquantitative, and quantitative assays. Qualitative assays (presence/absence) are encompassed by the invention wherein the simple presence of a histogram peak of mcf value above background (nonspecific binding) indicates the presence of analyte in the sample wherein the area of that histogram peak specifically identifies the analyte from a number of possible analytes as predetermined by the composition of the reagent. Semiquantitative assays (presence in the sample of analyte above a predetermined quantity or indirect measure of that quantity) are encompassed wherein the presence of a histogram peak above a certain predetermined mcf value (cut-off or discriminator) indicates the presence of the analyte in the sample at a concentration above a meaningful minimum and wherein the area of that histogram peak specifically identifies the analyte from a number of analytes as above. Using a similar identification logic, quantitative assays are described where the known concentration of the analyte(s) of interest as determined by other methods is related to a standard curve of fluorescence intensity such that the mcf value of the histogram curve are analytically related to concentration of analyte in the sample. These heretofore mentioned methods refer to discrete assays in which the analyte is specifically identified by the method utilizing the tautology of coated microsphere proportionality in the reagent with the area of the histogram peak. When these unique proportions are not employed in the reagent, a nondiscrete assay technology is derived wherein the presence or absence of one or more of the subject analytes can be stated but the analyte or analytes cannot be specifically identified.

## Claims

1. A composition comprising a reagent comprising:
a mixture of multiple discrete ligand complexes of ligand bound particulate supports, wherein each discrete ligand complex comprises a known, unique proportion of the composition and the sum of any two or more unique proportions also is unique as compared to every other possible proportion or sum of unique proportions of discrete ligand complexes in the composition; and
all the particulate supports of the composition are unlabelled and physically indistinguishable from each other.

2. A composition according to claim 1 wherein the unique proportions are chosen by reference to the formula n: 2n: 4n: 8n...2(^{m-1})n, where m equals the number of discrete ligand complexes and n is any number.

3. A composition according to claim 2 wherein m is 4 and n is 1.

4. A composition according to any preceding claim wherein the ligand specifically binds to an antigen or an antibody.

5. A composition according to any proceeding claim wherein the ligand is a biological macromolecule, a substance of abuse, a non-protein biological molecule or a pesticide.

6. A composition according to claim 5 wherein the ligand binds specifically to an analyte which is an antibody to HIV gp41, HIV p24, hepatitis B core protein or HTLV re-5.

7. A composition according to claim 5 wherein the ligand binds specifically to human IgG or human chorionic gonadotropin.

8. A composition according to any preceding claim wherein the particulate supports are microspheres.

9. A method of simultaneously determining the presence or absence of multiple analytes of interest in a sample, the method comprising:
(a) combining a sample with a composition as defined in any preceding claim to produce a reaction mixture containing specific binding pairs formed between a ligand and an analyte of interest;
(b) contacting the reaction mixture with a fluorescent labelling agent capable of binding to any specific binding pair present in the reaction mixture;
(c) removing any unbound fluorescent labelling agent from the reaction mixture;
(d) detecting the fluorescent signal from some or a preselected number of the particulate supports of the reaction mixture;
(e) obtaining a histogram plot of the particulate supports, or a preselected number thereof; and
(f) determining the presence or absence of the analytes of interest from the histogram plot.

10. A method according to claim 9 wherein the analytes of interest include antigens or antibodies.

## Patentansprüche

1. Zusammensetzung, umfassend ein Reagenz, das umfaßt:
ein Gemisch von vielen unterschiedlichen diskreten Ligandenkomplexen von Liganden-gebundenen Teilchenträgern, wobei jeder diskrete Ligandenkomplex einen einzigartigen bekannten Anteil der Zusammensetzung umfaßt und die Summe von zwei oder mehr einzigartigen Anteilen ebenfalls einzigartig ist im Vergleich zu jedem anderen möglichen Anteil oder der Summe von einzigartigen Anteilen von diskreten Ligandenkomplexen in der Zusammensetzung, und
wobei alle Teilchenträger der Zusammensetzung unmarkiert und physikalisch nicht voneinander unterscheidbar sind.

2. Zusammensetzung nach Anspruch 1, wobei die einzigartigen Anteile unter Bezug auf die folgende Formel ausgewählt werden n: 2n: 4n: 8n. .2(^{m-1})n, wobei m der Zahl an diskreten Ligandenkomplexen entspricht und n eine beliebige Zahl ist.

3. Zusammensetzung nach Anspruch 2, wobei m den Wert 4 und n den Wert 1 hat.

4. Zusammensetzung nach einem der vorangehenden Ansprüche, wobei der Ligand spezifisch an ein Antigen oder einen Antikörper bindet.

5. Zusammensetzung nach einem der vorangehenden Ansprüche, wobei der Ligand ein biologisches Makromolekül, ein mißbräuchlich angewendeter Stoff, ein biologisches Molekül, das kein Protein ist, oder ein Pestizid ist.

6. Zusammensetzung nach Anspruch 5, wobei der Ligand spezifisch an einen Analyten bindet, der ein Antikörper gegen HIV-gp41, HIV-p24, Hepatitis B Kernprotein oder HTLV-re-5 ist.

7. Zusammensetzung nach Anspruch 5, wobei der Ligand spezifisch an menschliches IgG oder menschliches Choriongonadotropin bindet.

8. Zusammensetzung nach einem der vorangehenden Ansprüche, wobei die Teilchenträger Mikrokügelchen sind.

9. Verfahren zur gleichzeitigen Bestimmung der Gegenwart oder Abwesenheit vieler unterschiedlicher interessierender Analyte in einer Probe, wobei das Verfahren umfaßt:
(a) die Kombination einer Probe mit einer Zusammensetzung gemäß der Definition in einem der vorangehenden Ansprüche zur Herstellung eines Reaktionsgemisches, das spezifische Bindungspaare enthält, die zwischen einem Liganden und einem interessierenden Analyten gebildet wurden;
(b) das Inkontaktbringen des Reaktionsgemisches mit einem fluoreszierenden Markierungsreagenz, das an ein beliebiges spezifisches Bindungspaar binden kann, das in dem Reaktionsgemisch vorliegt;
(c) das Entfernen von ungebundenem fluoreszierenden Markierungsreagenz aus dem Reaktionsgemisch;
(d) den Nachweis des Fluoreszenzsignals von einigen oder einer ausgewählten Zahl der Teilchenträger in dem Reaktionsgemisch;
(e) die Gewinnung einer Histogrammdarstellung der Teilchenträger oder einer ausgewählten Zahl davon; und
(f) die Bestimmung der Gegenwart oder Abwesenheit der interessierenden Analyten aus der Histogrammdarstellung.

10. Verfahren nach Anspruch 9, wobei die interessierenden Analyte Antigene oder Antikörper einschließen.

## Revendications

1. Une composition comprenant un réactif comprenant :
un mélange de complexes de multiples ligands discrets constitués de supports particulaires liés à des ligands, où chaque complexe de ligands discrets comprend une proportion unique connue de la composition et la somme de deux ou plusieurs proportions uniques quelconques est également unique par comparaison à toute autre proportion possible ou somme de proportions uniques de complexes de ligands discrets dans la composition ; et
tous les supports particulaires de la composition sont non marqués et physiquement impossibles à distinguer les uns des autres.

2. Une composition selon la revendication 1 dans laquelle les proportions uniques sont choisies en référence à la formule n : 2n : 4n : 8n...2(^{m-1})n, où m est égal au nombre de complexes de ligands discrets et n est un nombre quelconque.

3. Une composition selon la revendication 2 dans laquelle m est 4 et n est 1.

4. Une composition selon l'une quelconque des revendications précédentes dans laquelle le ligand se lie spécifiquement à un antigène ou à un anticorps.

5. Une composition selon l'une quelconque des revendications précédentes dans laquelle le ligand est une macromolécule biologique, une substance utilisée comme drogue, une molécule biologique non protéique ou un pesticide.

6. Une composition selon la revendication 5 dans laquelle le ligand se lie spécifiquement à un analyte qui est un anticorps dirigé contre gp41 de HIV, p24 de HIV, une protéine de core de l'hépatite B ou re-5 de HTLV.

7. Une composition selon la revendication 5 dans laquelle le ligand se lie spécifiquement à une IgG humaine ou à une gonadotropine chorionique humaine.

8. Une composition selon l'une quelconque des revendications précédentes dans laquelle les supports particulaires sont des microsphères.

9. Une méthode de détermination simultanée de la présence ou de l'absence de multiples analytes d'intérêt dans un échantillon, la méthode comprenant les étapes consistant à :
(a) combiner un échantillon avec une composition telle que définie dans l'une quelconque des revendications précédentes pour produire un mélange réactionnel contenant des paires de liaison spécifiques formées entre un ligand et un analyte d'intérêt ;
(b) mettre en contact le mélange réactionnel avec un agent de marquage fluorescent capable de se lier à une quelconque paire de liaison spécifique présente dans le mélange réactionnel ;
(c) retirer tout agent de marquage fluorescent non lié du mélange réactionnel ;
(d) détecter le signal fluorescent de certains ou d'un nombre présélectionné de supports particulaires du mélange réactionnel ;
(e) obtenir un histogramme des supports particulaires, ou d'un nombre présélectionné de ceux-ci ; et
(f) déterminer la présence ou l'absence des analytes d'intérêt à partir de l'histogramme.

10. Une méthode selon la revendication 9 dans laquelle les analytes d'intérêt comprennent des antigènes ou des anticorps.
